# EUROPEAN PATENT APPLICATION

(11) **EP 1 408 112 A1**
(43) Date of publication of application: **14.04.2004**
(21) Application number: 02733464.8
(22) Date of filing: 11.06.2002
(51) Int. Cl.: C12N 15/12, C12N 15/08, C07K 16/40, C12N 5/20, A61K 39/395, A61P 29/00, A61P 35/00, A61P 43/00, G01N 33/53

(54) **MONOCLONAL ANTIBODY BINDING TO MT4−MMP CATALYTIC DOMAIN**

(30) Priority: 11.06.2001 JP 2001176256
(71) Applicant: KYOWA HAKKO KOGYO CO., LTD., Chiyoda-ku, Tokyo 100-8185 (JP)
(72) Inventor: MIKI, Ichiro, Kyowa Hakko Kogyo Co., Ltd, Sunto-gun, Shizuoka 411-8731 (JP); OHTA, So, Kyowa Hakko Kogyo Co., Ltd, Machida-shi, Tokyo 194-8533 (JP); SHITARA, Kenya, Kyowa Hakko Kogyo Co., Ltd, Machida-shi, Tokyo 194-8533 (JP); FURUYA, Akiko, Kyowa Hakko Kogyo Co., Ltd, Machida-shi, Tokyo 194-8533 (JP)
(74) Representative: VOSSIUS & PARTNER
(86) International application number: PCT/JP2002/005788
(87) International publication number: WO 2002/101046

(57) **Abstract**

The present invention relates to a monoclonal antibody which specifically reacts with natural MT4-MMP or solubilized MT4-MMP. Also, the present invention provides a monoclonal antibody which specifically and efficiently recognizes a natural MT4-MMP catalytic domain; a human chimeric antibody, a CDR-grafted antibody, a single chain antibody and a disulfide stabilized antibody, comprising the same; and a method for detecting and determining MT4-MMP protein using the antibodies. Furthermore, the present invention provides a diagnostic method, a diagnostic agent and a therapeutic agent for various diseases relating to MP4-MMP such as inflammation and cancer, using the antibodies.

## Description

### TECHNICAL FIELD

The present invention relates to a monoclonal antibody which specifically reacts with an MT4-MMP catalytic domain. Furthermore, the present invention relates to a diagnostic method, a diagnostic agent, a therapeutic agent, a reagent and a kit for diseases relating to MP4-MMP such as inflammation and cancer, using the monoclonal antibody.

### BACKGROUND OF THE INVENTION

Matrix metalloproteinase (MMP) is an enzyme which hydrolyzes extracellular matrix-constituting proteins and is produced from markedly varied cell such as connective tissue cells, epithelial cells, leukocytes and cancer cells. It is considered that MMP is related in phenomena which are important for maintaining life such as development, differentiation, tissue formation and tissue restoration under physiological conditions, and is also related in various diseases which accompany tissue destruction such as arthritis and glomerulonephritis and in metastasis and infiltration of cancer cells. MMP is a group of proteases which have Zn²⁺ in the active center and show certain characteristics, e.g., they are produced as non-active precursor propeptides and later activated in extracellular regions, and MMPs exceeding 20 species have so far been reported [*J*. *Clin*. *Oncol*., 18, 1135-1149 (2000)].

Membrane type-matrix metalloproteinases (MT-MMP) are members of the MMP gene family which have recently been discovered in succession, and the presence of 6 species (MT1-MMP, MT2-MMP, MT3-MMP, MT4-MMP, MT5-MMP and MT6-MMP) have so far been reported. Each of them has the propeptide domain, active domain and hemopexin domain structures common in MMP, and also has a hydrophobic region on the C-terminal end considered to be a transmembrane domain which is not present in MMPs other than MT-MMP. Though there are many unclear point regarding their functions, MT1-MMP (MMP-14), MT2-MMP (MMP-15), MT3-MMP (MMP-16) and MT5-MMP (MMP-24) have relatively high homology of the amino acid sequence in active domain, and it has been reported that each of them has an activity to convert pro-MMP-2 into mature MMP-2 [*Eur*. *J*. *Biochem*., 231, 602-608 (1995); *J*. *Biol*. *Chem*., 274, 8925-8932 (1999)], so that it can be considered also that they are positioned in the upstream of the MMP cascade. On the other hand, MT4-MMP (MMP-17) and MT6-MMP (MMP-25) have high homology regarding their amino acid sequences but have low homology with MT1-MMP, MT2-MMP, MT3-MMP and MT5-MMP. Thus, it is considered that MT4-MMP (MMP-17) and MT6-MMP (MMP-25) do not induce activation of the pro-MMP-2 [*J*. *Biol*. *Chem*., 275, 14046-14055 (2000); *Cancer Res.,* 60, 877-882 (2000)]. Moreover, it has been reported that though MT4-MMP and MT6-MMP have the hydrophobic region considered to be a transmembrane domain, they lack in the cytoplasmic domain structure which is composed of 20 to 23 amino acids and found in MT1-MMP, MT2-MMP, MT3-MMP and MT5-MMP, and they exist as a glycosyl-phosphatidyl inositol (GPI) anchor type existing form. Accordingly, it is considered that MT4-MMP and MT6-MMP are different from known species of MMP or MT-MMP in terms of not only their amino acid sequences but also their existing forms [*J*. *Biol*. *Chem*., 274, 34260-34266 (1999); *FEBSLett.,* 480, 142-146 (2000)].

Although expression of MT4-MMP has so far been found frequently in human leukocytes by the expression analysis of mRNA by Northern blotting, it has been found that it is expressed in other tissues but limiting to the brain, the colon, ovaries and testes [*Cancer Res.,* 56, 944-949 (1996)]. Moreover, since it has been reported that expression of the mRNA was found also in various tumor cell lines and normal fibroblast cell lines [*Matrix Biol*., 18, 145-148 (1999); WO00/18805], it is considered that this enzyme is concerned in various diseases with tissue destruction and also in metastasis and infiltration of cancer cells.

An antibody having high antigen specificity and high affinity is considerably important for examining function and expression of a specific protein in a cell or tissue. A monoclonal antibody prepared by using, as the immunogen, a hemopexin domain-containing amino acid sequence of positions 321 to 550, which corresponds to the amino acid sequence of positions 335 to 526 described in SEQ ID NO:6, is known as an antibody for MT4-MMP (WO00/18805). The antibody is useful for detection under denaturing conditions such as Western blotting and detection of cells of a protein expressing a large amount of protein such as MT4-MMP transfectants: However, reactivity of the antibody is considerably weak against natural MT4-MMP expressing on general cell lines or leukocyte or against solubilized MT4-MMP.

### DISCLOSURE OF THE INVENTION

An object of the present invention is to provide a monoclonal antibody having reactivity to natural MT4-MMP which is expressed on a cell line or leukocyte and solubilized MT4-MMP which is present in a tissue or serum. The monoclonal antibody of the present invention is useful for diagnosis or treatment of diseases relating to MT4-MMP.

The present invention relates to the following (1) to (32).
(1) A monoclonal antibody which specifically binds to an MT4-MMP catalytic domain.
(2) The monoclonal antibody according to the above (1), wherein the MT4-MMP catalytic domain is an amino acid sequence comprising 128th to 296th positions in the amino acid sequence represented by SEQ ID NO:6.
(3) The monoclonal antibody according to the above (1) or (2), wherein the monoclonal antibody is a monoclonal antibody produced by a hybridoma selected from the group consisting of hybridoma KM2895, hybridoma KM2896, hybridoma KM2897 and hybridoma KM2904.
(4) A hybridoma which produces the monoclonal antibody according to any one of the above (1) to (3).
(5) The hybridoma according to the above (4), wherein the hybridoma is a hybridoma selected from the group consisting of hybridoma KM2895, hybridoma KM2896, hybridoma KM2897 and hybridoma KM2904.
(6) The monoclonal antibody according to any one of the above (1) to (3), wherein the monoclonal antibody is a recombinant antibody.
(7) The monoclonal antibody according to the above (6), wherein the recombinant antibody is a monoclonal antibody selected from a humanized antibody and an antibody fragment.
(8) The monoclonal antibody according to. the above (7), wherein the humanized antibody is a human chimeric antibody.
(9) The human chimeric antibody according to the above (8), which comprises an antibody heavy chain (H chain) variable region (V region) and an antibody light chain (L chain) V region of the monoclonal antibody according to any one of the above (1) to (3) and an H chain constant region (C region) and an L chain C region of a human antibody.
(10) The human chimeric antibody according to the above (9), wherein the amino acid sequences of the H chain V region and L chain V region have the same amino acid sequences of the H chain V region and L chain V region, respectively, of a monoclonal antibody selected from the group consisting of monoclonal antibodies KM2895, KM2896, KM2897 and KM2904.
(11) The monoclonal antibody according to the above (7), wherein the humanized antibody is a complementarity determining region-grafted antibody (CDR-grafted antibody).
(12) The CDR-grafted antibody according to the above (11), which comprises complementarity determining regions of H chain and L chain V regions of the monoclonal antibody according to any one of the above (1) to (3) and H chain and L chain C regions and a framework region of a V region of a human antibody.
(13) The CDR-grafted antibody according to the above (12), wherein the amino acid sequences of the H chain V region and the L chain V region have the same amino acid sequences of an H chain V region and an L chain V region, respectively, of a monoclonal antibody selected from the group consisting of monoclonal antibodies KM2895, KM2896, KM2897 and KM2904.
(14) The monoclonal antibody according to the above (7), wherein the antibody fragment is an antibody selected from the group consisting of Fab, Fab', F(ab')₂, a single chain antibody and a disulfide-stabilized antibody.
(15) The single chain antibody according to the above (14), which comprises an H chain V region and an L chain V region of the monoclonal antibody according to any one of the above (1) to (3).
(16) The single chain antibody according to the above (15), wherein the amino acid sequences of the H chain V region and the L chain V region of the single chain antibody have the same amino acid sequences of an H chain V region and an L chain V region, respectively, of a monoclonal antibody selected from the group consisting of monoclonal antibodies KM2895, KM2896, KM2897 and KM2904.
(17) The disulfide-stabilized antibody according to the above (14), which comprises an H chain V region and an L chain and V region of the monoclonal antibody according to any one of the above (1) to (3).
(18) The disulfide-stabilized antibody according to the above (17), wherein the amino acid sequences of the H chain V region and the L chain V region of the disulfide-stabilized antibody have the same amino acid sequences of an H chain V region and an L chain V region, respectively, of a monoclonal antibody selected from the group consisting of monoclonal antibodies KM2895, KM2896, KM2897 and KM2904.
(19) The monoclonal antibody according to any one of the above (1) to (3) and (6) to (18), wherein the monoclonal antibody is a fusion antibody linked with an agent chemically or genetically.
(20) A method for immunologically detecting an MT4-MMP catalytic subunit, which comprises using the monoclonal antibody according to any one of the above (1) to (3) and (6) to (19).
(21) The method according to the above (20), wherein the immunologically detecting method is selected from the group consisting of immunoassay, Western blotting, immunohistochemical staining, cell immunostaining and dot blotting.
(22) A method for immunologically determining an MT4-MMP catalytic subunit, which comprises using the monoclonal antibody according to any one of the above (1) to (3) and (6) to (19).
(23) The method according to the above (22), wherein the immunologically detecting method is a method selected from the group consisting of immunoassay, Western blotting, immunohistochemical staining, cell immunostaining and dot blotting.
(24) A method for diagnosing diseases relating to MT4-MMP, which comprises using the monoclonal antibody according to any one of the above (1) to (3) and (6) to (19).
(25) The diagnostic method according to the above (24), wherein the disease relating to MT4-MMP is rheumatoid arthritis.
(26) An agent for diagnosing disease relating to MT4-MMP, which comprises the monoclonal antibody according to any one of the above (1) to (3) and (6) to (19) as an active ingredient.
(27) The diagnostic agent according to the above (26), wherein the disease relating to MT4-MMP is rheumatoid arthritis.
(28) A therapeutic agent for treating diseases relating to MT4-MMP, which comprises the monoclonal antibody according to any one of the above (1) to (3) and (6) to (19) as an active ingredient.
(29) The therapeutic agent according to the above (28), wherein the disease relating to MT4-MMP is rheumatoid arthritis.
(30) A reagent which comprises the monoclonal antibody according to any one of the above (1) to (3) and (6) to (19).
(31) A kit for detecting diseases relating to MT4-MMP, which comprises the reagent according to the above (30).
(32) The kit according to the above (31), wherein the disease relating to MT4-MMP is rheumatoid arthritis.

The monoclonal antibody which specifically binds to an MT4-MMP catalytic domain of the present invention may be any monoclonal antibody, so long as it specifically binds to an MT4-MMP catalytic domain. It is preferably a monoclonal antibody which specifically binds to an MT4-MMP catalytic domain comprising 128th to 296th positions of the MT4-MMP amino acid sequence represented by SEQ ID NO:6.

The monoclonal antibody includes an antibody produced by a hybridoma and a recombinant antibody produced by a transformant transformed with an antibody gene-containing expression vector.

That is, an anti-MT4-MMP catalytic domain monoclonal antibody can be obtained by preparing an MT4-MMP catalytic domain protein, a peptide chemically synthesized based on the amino acid sequence of an MT4-MMP catalytic domain protein (WO00/18805) or the like, as an antigen, inducing an antibody-producing cell having the antigen specificity from an animal immunized with the antigen, preparing a hybridoma by fusing it with a myeloma cell, and then culturing the hybridoma; or by administering the hybridoma cells to an animal to cause ascitic tumor, and separating and purifying the culture medium or ascitic fluid.

The recombinant antibody of the present invention is a product obtained by modifying the above monoclonal antibody of the present invention using gene recombination techniques. The recombinant antibody includes antibodies produced by gene recombination techniques such as a humanized antibody and an antibody fragment. Among these recombinant antibodies, those which have characteristics of a monoclonal antibody, low antigenicity and prolonged blood half-life are preferable as therapeutic agents. The humanized antibody includes a human chimeric antibody, a human CDR (complementary determining region; hereinafter referred to as "CDR")-grafted antibody and the like.

The antibody fragment of the present invention includes Fab (abbreviation of fragment of antigen binding), Fab', F(ab')₂, a single chain antibody (single chain Fv, hereinafter referred to as "scFv") and a disulfide stabilized antibody (disulfide stabilized Fv, hereinafter referred to as "dsFv").

A human chimeric antibody is an antibody which comprises a non-human antibody heavy chain variable region (hereinafter referred to as "VH") and a non-human antibody light chain variable region (hereinafter referred to as "VL"), and a human antibody heavy chain constant region (hereinafter referred to as "CH") and a human antibody light chain constant region (hereinafter referred to as "CL").

The human chimeric antibody of the present invention can be produced by obtaining cDNAs encoding VH and VL from a hybridoma which produces a monoclonal antibody which specifically binds to an MT4-MMP catalytic domain, inserting them into an expression vector for host cell having genes encoding human antibody CH and human antibody CL to thereby construct a human chimeric antibody expression vector, and then introducing the vector into a host cell to express the human chimeric antibody of the present invention on the host cell.

The structure of the C region of the human chimeric antibody of the present invention may be one belonging to any immunoglobulin (hIg) class, is preferably the C region of immunoglobulin of IgG class, and more preferably of IgG1, IgG2, IgG3, IgG4 and the like belonging to IgG class.

A human CDR-grafted antibody is an antibody in which CDRs of VH and VL of a human antibody are respectively substituted with CDR sequences of an antibody derived from a non-human animal.

The human CDR-grafted antibody of the present invention can be produced by constructing cDNAs encoding V regions in which CDR sequences of VH and VL of a human antibody are replaced with CDR sequences of VH and VL of an antibody which specifically binds to an MT4-MMP catalytic domain derived from a non-human animal, inserting the cDNAs into an expression vector for host cell having genes encoding human antibody CH and human antibody CL to thereby construct a human CDR-grafted antibody expression vector, and then introducing the expression vector into a host cell to express the human CDR-grafted antibody of the present invention on the host cell.

The structure of the C region of the human CDR-grafted antibody may be one belonging to any immunoglobulin (hIg) class, is preferably the C region of immunoglobulin of IgG class, and more preferably of IgG1, IgG2, IgG3, IgG4 and the like belonging to IgG class.

An Fab is a fragment having a molecular weight of about 50,000 and antigen binding activity, which comprises about a half of the N-terminal side of H chain and the entire L chain obtained by digesting the upper peptide part of two disulfide bonds crosslinking two H chains in the hinge region of IgG with an enzyme, papain.

The Fab of the present invention can be obtained by treating an antibody which specifically binds to an MT4-MMP catalytic domain with a papain. Furthermore, the Fab can be produced by inserting DNA encoding an Fab fragment of the antibody into an expression vector for host cell, and introducing the vector into a host cell to express the Fab of the present invention on the host cell.

An Fab' is a fragment having a molecular weight of about 50,000 and antigen binding activity, which is obtained by cutting a disulfide bond of the hinge region of the F(ab')₂.

The Fab' of the present invention can be obtained by treating an antibody which specifically binds to an MT4-MMP catalytic domain with a reducing agent, dithiothreitol. Furthermore, the Fab' can be produced by inserting DNA encoding an Fab' fragment of the antibody into an expression vector for host cell, and introducing the vector into a host cell to express the Fab' of the present invention on the host cell.

An F(ab')₂ is a fragment having a molecular weight of about 100,000 and antigen binding activity, which comprises two Fabs bound via the hinge region obtained by digesting the lower part of two disulfide bonds crosslinking two H chains in the hinge region of IgG with an enzyme, trypsin.

The F(ab')₂ of the present invention can be obtained by treating an antibody which specifically binds to an MT4-MMP catalytic domain with a trypsin. Furthermore, it can be produced by inserting DNA encoding an F(ab')₂ fragment of the antibody into an expression vector for host cell, and introducing the vector into a host cell to express the F(ab')₂ of the present invention on the host cell.

A single chain antibody (scFv) is a VH-P-VL or VL-P-VH polypeptide in which one chain VH and one chain VL are linked using an appropriate peptide linker (hereinafter referred to as "P"). The VH and VL in the scFv of the present invention may be any of the monoclonal antibody and human CDR-grafted antibody of the present invention.

The scFv of the present invention can be produced by obtaining cDNAs encoding VH and VL from a hybridoma or transformant capable of producing an antibody which specifically binds to an MT4-MMP catalytic domain, constructing an expression vector for the single chain antibody, followed by inserting the cDNA into the expression vector and introducing the expression vector into a host cell to express the scFv.

A disulfide stabilized antibody (dsFV) is obtained by binding polypeptides in which one amino acid residue of each of VH and VL is substituted with a cysteine residue via a disulfide bond between the cysteine residues. The amino acid residue to be substituted with a cysteine residue can be selected based on a three-dimensional structure estimation of the antibody in accordance with the method described by Reiter *et al*. [*Protein Engineering, 7*: 697 (1994)]. The VH and VL contained in the disulfide stabilized antibody of the present invention may be any of a monoclonal antibody or a human CDR-grafted antibody.

The disulfide stabilized antibody of the present invention can be produced by obtaining cDNAs encoding VH and VL from a hybridoma or transformant capable of producing an antibody which specifically binds to an MT4-MMP catalytic domain, inserting the cDNA into an appropriate expression vector and introducing the expression vector into a host cell to express the disulfide stabilized antibody.

A fusion antibody is obtained by linking the above antibody with an agent chemically or genetically.

The agent may be any of a radioisotope, a protein, a low molecular weight molecule and the like.

When the fusion antibody is used as a diagnostic agent, the agent includes a label used in immunoassay. The label includes an enzyme such as alkaline phosphatase, peroxidase and luciferase, a luminescent substance such as acridinium ester and rofin, a fluorescent substance such as FITC and RITC, and the like.

The fusion antibody of the present invention can be produced by linking an antibody which specifically binds to an MT4-MMP catalytic domain with an agent chemically. Also, when the agent is a protein, the fusion antibody can be produced by binding a cDNA encoding the protein to a cDNA encoding the antibody, inserting the cDNA into an appropriate expression vector, and introducing the expression vector into a host cell to express the fusion antibody.

The present invention is explained below in detail.

### 1. Preparation of anti-MT4-MMP (also referred to as "MMP-17") catalytic domain monoclonal antibody

### (1) Preparation of antigen

The antigen includes a cell expressing an MT4-MMP catalytic domain intracellularly or a fraction thereof, an MT4-MMP catalytic domain protein or a partial protein of an MT4-MMP catalytic domain, and a fusion protein of the protein with the Fc region of an antibody.

The cell expressing an MT4-MMP catalytic domain includes U937 (human histiocytic lymphoma), THP-1 (human monocyte), Jurkat (human acute T cell leukemia) and the like (WO00/18805). Although the cell can be used directly as the antigen, an MT4-MMP catalytic domain fractionated from the cell using an ordinary enzyme separation and purification method which will be described later can also be used as the antigen.

Furthermore, an MT4-MMP catalytic domain protein, a partial protein of MT4-MMP catalytic domain, a fusion protein of said protein with the Fc region of an antibody or the like can be expressed and used as the antigen by preparing a DNA encoding an MT4-MMP catalytic domain from the above cell using genetic engineering techniques. The method is described as follows.

In order to obtain a DNA encoding an MT4-MMP catalytic domain, a cDNA library is prepared from the cDNA described in WO00/18805 or the above cell expressing an MT4-MMP catalytic domain by the conventional method [*Molecular Cloning,* 2nd edition, Cold Spring Harbor Lab. Press New York (1989), hereinafter referred to as *"Molecular Cloning,* 2nd edition" and *Current Protocols in Molecular Biology,* Supplement 1-38, hereinafter referred to as "*Current Protocols*"].

That is, mRNA is extracted, and cDNA is synthesized from the mRNA. A cDNA library is prepared by inserting the thus obtained cDNA into a cloning vector and introducing the vector into a host cell. A DNA coding for the MT4-MMP catalytic domain can be obtained by selecting a transformant comprising the cDNA of interest from the library.

The method for preparing a total RNA from a cell expressing the MT4-MMP catalytic domain includes the guanidine/cesium chloride method and guanidine thiocyanate method [*Methods in Enzymol*., 154, 3 (1987)] and the like. Also, the method for preparing mRNA from a total RNA includes a column method or batch method using oligo dT cellulose or the like. In addition, the mRNA can also be prepared by using a kit such as Fast Track mRNA Isolation Kit (manufactured by Invitrogen) or Quick Prep mRNA Purification Kit (manufactured by Pharmacia).

The method for synthesizing cDNA from the mRNA obtained in the above includes Okayama-Berg method [*Mol*. *Cell*. *Biol*., 2, 161 (1982), Gubler-Hoffman method [*Gene,* 25, 263 (1983)] and the like. Furthermore, the cDNA can also be synthesized by using a kit such as Superscript Plasmid System for cDNA Synthesis and Plasmid Cloning (manufactured by Gibco BRL) or Zap-cDNA Synthesis Kit (manufactured by Stratagene).

As the cloning vector for insertion of cDNA, any vector such as a phage vector or a plasmid vector can be used, so long as it is autonomously replicable in a host cell. Examples include ZAP Express [manufactured by STRATAGENE, *Strategies*, 5, 58 (1992)], pBluescript II SK(+) *[Nucleic Acids Research,* 17, 9494 (1989)], λZAP II (manufactured by STRATAGENE), λgt10 and λgt11 [*DNA Cloning*, *A Practical Approach*, 1, 49 (1985)], λTriplEx (manufactured by Clontech), λExCell (manufactured by Pharmacia), pT7T3 18U (manufactured by Pharmacia), pcD2 [*Mol*. *Cell*. *Biol*., 3, 280 (1983)], pUC18 [*Gene,* 33, 103 (1985)], pAMo [*J*. *BIol*. *Chem*., 268, 22782 (1993), also known as pAMoPRCsSc (Japanese Published Unexamined Patent Application No. 336963/93)] and the like.

Any microorganism can be used as the host microorganism, so long as the microorganism belongs to *Escherichia coli.* Examples include *Escherichia coli* XL1-Blue MRF' [manufactured by Stratagene, *Strategies,* 5, 81 (1992)], *Escherichia coli* C600 [*Genetics,* 39, 440 (1954)], *Escherichia coli* YI088 [*Science*, 222, 778 (1983)], *Escherichia coli* YIO90 [*Science*, 222, 778 (1983)], *Escherichia coli* NM522 [*J*. *Mol. Biol*., 148, 427-448 (1981)], JM105 [*Gene*, 38, 275 (1985)], *Escherichia coli* SOLRTM Strain (commercially available from Stratagene), *Escherichia coli* LE392 *(Molecular Cloning,* 2nd edition) and the like

A cDNA library is prepared by inserting cDNA into the above cloning vector and introducing the cloning vector into a host cell.

When the cloning vector is a plasmid, introduction into a host cell is carried out by electroporation, the calcium chloride method or the like. When the cloning vector is a phage, introduction into a host cell is carried out by the *in vitro* packaging method or the like.

In order to obtain a transformant comprising DNA encoding an MT4-MMP catalytic domain from the thus obtained cDNA library, for example, a probe is prepared based on the nucleotide sequence of DNA encoding an MT4-MMP catalytic domain described in WO00/18805, and the probe is labeled with a fluorescent substance, radiation, an enzyme or the like, and plaque hybridization, colony hybridization, Southern hybridization and the like are carried out to select a hybridizable transformant.

A recombinant vector is constructed by inserting a full length or partial fragment cDNA encoding the thus obtained MT4-MMP catalytic domain into downstream of a promoter of an appropriate vector. An MT4-MMP catalytic domain-expressing cell is obtained by introducing the recombinant vector into a host cell is cultured in an appropriate medium to thereby produce a full length or partial fragment of the MT4-MMP catalytic domain as it is or as a fusion protein intracellularly or in culture supernatant.

As the host, any of a bacterium, yeast, an animal cell, an insect cell and the like can be used, so long as the gene of interest can be expressed. The bacterium includes bacteria belonging to the genus *Escherichia*, the genus *Bacillus* and the like such as *Escherichia coli* and *Bacillus subtilis.* The yeast includes *Saccharomyces cerevisiae*, *Schizosaccharomyces pombe* and the like. The animal cell includes human cell Namalwa cell, monkey cell COS cell, Chinese hamster cell CHO cell, and the like. The insect cell includes Sf9 and Sf21 (manufactured by PharMingen), High Five (manufactured by Invitrogen), and the like.

As the vector into which the DNA of the present invention is inserted, any vector can be used, so long as the DNA can be inserted and can be expressed in a host cell. When a bacterium such as *Escherichia coli* is used as a host, the expression vector is preferably constructed with a promoter, a ribosome binding sequence, the DNA of the present invention, a transcription termination sequence, and optionally a promoter-controlling gene. Examples include commercially available pGEX (manufactured by Pharmacia), pET system (manufactured by Novagen) and the like.

As the method for introducing the recombinant vector into a bacterium, any method can be used, so long as it is a method for introducing a DNA into a bacterium. Examples include the method using a calcium ion [*Proc*. *Natl*. *Acad*. *Sci*., *USA*, 69, 211 (1972)], the protoplast method [Japanese Published Unexamined Patent Application No. 248394/88] and the like. When a yeast is used as a host, the expression vector includes YEp13 (ATCC37115), YEp24 (ATCC37051), YCp50 (ATCC37419) and the like.

As the method for introducing the recombinant vector into a yeast, any method can be used, so long as it is a method for introducing a DNA into a yeast. Examples include electroporation [*Methods*. *Enzymol*., 194, 182 (1990)], the spheroplast method [*Proc*. *Natl*. *Acad*. *Sci*. *USA*, 84, 1929 (1978)], the lithium acetate method [*J*. *Bacteriol*., 153, 163 (1983)] and the like.

When an animal cell is used as the host, the expression vector includes pAGE107 [Japanese Published Unexamined Patent Application No. 22979/91; *Cytotechnology*, 3, 133 (1990)], pAGE103 [*J*. *Biochemistry*, 101, 1307 (1987)] and the like.

Any promoter can be used, so long as it can be expressed in an animal cell. Examples include a promoter of IE (immediate early) gene of cytomegalovirus, a promoter of SV40 or metallothionein, and the like. Also, the enhancer of IE gene of human CMV can be used together with the promoter.

As the method for introducing the recombinant vector into an animal cell, any method can be used, so long as it is a method for introducing a DNA into an animal cell. Examples include electroporation [*Cytotechnology*, 3, 133 (1990)], the calcium phosphate method (Japanese Published Unexamined Patent Application No. 227075/90), the lipofection method [*Proc*. *Natl*. *Acad*. *Sci*. *USA*, 84, 7413 (1987)] and the like.

When an insect cell is used as the host, a protein can be expressed by the method described in, for example, *Current Protocols* (Supplements 1-34), *Bacurovirus Expression Vectors*, *A Laboratory Manual*, or the like. Specifically, a recombinant gene transfer vector and baculovirus described below are co-transfected into an insect cell to obtain a recombinant virus in an insect cell culture supernatant, and then the insect cell is infected with the resulting recombinant virus to obtain a protein-expressing insect cell.

The gene transfer vector includes pVL1392, pVL1393, pBlueBacIII (all manufactured by Invitrogen) and the like.

The bacurovirus includes *Autographa californica* nuclear polyhedrosis virus which infects insects of the family *Noctuidae*, and the like.

The method for co-transfecting the above recombinant gene transfer vector and the above bacurovirus for the preparation of the recombinant virus includes the calcium phosphate method (Japanese Published Unexamined Patent Application No. 227075/90), the lipofection method [*Proc*. *Natl*. *Acad Sci*. *USA*, 84, 7413 (1987)] and the like.

Furthermore, a recombinant bacurovirus is prepared by using Bacuro Gold Starter Kit manufactured by PharMingen or the like and then a protein can be produced by infecting an insect cell such as the above Sf9, Sf21 or High Five with the recombinant virus [*Bio*/*Technology*, 6, 47 (1988)].

As a method for expressing a gene, secretory production, fusion protein production and the like are being developed in addition to direct expression, and any of the methods can be used. For example, it can be carried out according to the method described in *Molecular Cloning,* 2nd Edition.

The protein to be fused includes β-galactosidase, protein A, IgG binding region of protein A, chloramphenicol acetylransferase, poly(Arg), poly(Glu), protein G, maltose binding protein, glutathione S-transferase, polyhistidine chain (His-tag), S peptide, DNA binding protein domain, Tac antigen, thioredoxin, green fluorescent protein, epitope of any antibody, and the like [Akio Yamakawa, *Experimental Medicine (Jikken Igaku)*, 13, 469-474 (1995)].

A full length or partial fragment of an MT4-MMP catalytic domain can be produced as it is or as a fusion protein by culturing the thus obtained transformant in a medium to form and accumulate a full length or partial fragment of an MT4-MMP catalytic domain as it is or as a fusion protein in the culture, and recovering it from the culture.

Culturing of the transformant of the present invention in a medium is carried out by the ordinary used method in culturing of the host.

As the medium for culturing a transformant obtained using a microorganism such as *Escherichia coli* or yeast as the host, the medium may be either a natural medium or a synthetic medium, so long as it comprises materials such as a carbon source, a nitrogen source, an inorganic salt and the like which can be assimilated by the microorganism and culturing of the transformant can be efficiently carried out *(Molecular Cloning,* 2nd edition). The culturing is carried out generally under aerobic conditions such as shaking culture or submerged-aeration stirring culture at 15 to 40°C for 16 hours to 96 hours. During the culturing, the pH is maintained at 3.0 to 9.0. The pH is adjusted using an inorganic or organic acid, an alkali solution, urea, calcium carbonate, ammonia or the like. If necessary, an antibiotic such as ampicillin or tetracycline can be added to the medium during the culturing.

When a transformant obtained using an animal cell as the host is cultured, the medium includes generally used RPMI 1640 medium, Eagle's MEM medium, the media to which fetal calf serum, *etc.* is added, and the like. The culturing is carried out generally at 35 to 37°C for 3 to 7 days in the presence of 5% CO₂. If necessary, an antibiotic such as kanamycin or penicillin can be added to the medium during the culturing.

When a transformant obtained using an insect cell as the host is cultured, the medium includes generally used TNM-FH medium (manufactured by PharMingen), Sf900 II SFM medium (manufactured by Life Technologies), ExCell 400 and ExCell 405 (both manufactured by JRH Biosciences) and the like. The culturing is carried out generally at 25 to 30°C for 1 to 4 days. If necessary, an antibiotic such as gentamicin can be added to the medium during the culturing.

In the above, when culturing is carried out with a serum-free medium for animal cell or insect cell, a full length or partial fragment of an MT4-MMP catalytic domain obtained as it is or as a fusion protein can be easily purified. Thus, a serum-free medium is preferred.

When a full length or partial fragment of an MT4-MMP catalytic domain is accumulated as it is or as a fusion protein in the host cell, cells are centrifuged after the culturing and suspended in an aqueous buffer, and then disrupted using ultrasonic oscillator, French press or the like, and a protein is recovered from the supernatant obtained by centrifugation.

Also, when insoluble body is formed intracellularly, a protein can be made into three-dimensional structure by diluting or dialyzing the solubilized solution in at such a concentration of the protein denaturing agent that does not denature protein or without the protein denaturing agent, after solubilized with a protein denaturing agent.

When a full length or partial fragment of an MT4-MMP catalytic domain is extracellularly secreted as it is or as a fusion protein, the expressed protein can be recovered from the culture supernatant. Isolation and purification can be carried out by separation operation such as solvent extraction, fractional precipitation by an organic solvent, salting-out, dialysis, centrifugation, ultra filtration, ion exchange chromatography, gel filtration chromatography, hydrophobic chromatography, affinity chromatography, reverse-phase chromatography, crystallization and electrophoresis alone or as a combination thereof.

Alternatively, a protein partial sequence of 5 to 30 residues is selected as the polypeptide having partial sequence. In order to obtain an antibody which recognizes said protein having natural structure, a partial sequence existing on the surface of the protein in its three-dimensional structure is selected as an antigen peptide. As the method for predicting the partial sequence existing on the surface of the protein in its three-dimensional structure, commercially available protein sequence analyzing software such as Genetyx Mac can be exemplified. Generally, a moiety having low hydrophilic region is present inside of protein in view of its three-dimensional structure in many cases, and a moiety having high hydrophilic region is present on the protein surface in many cases. In addition, N-terminus and C-terminus of protein are present on the protein surface in many cases. However, a partial peptide selected in this manner does not always become an antigen which establishes the antibody of interest.

In order to crosslink a partial peptide with a carrier protein which will be described later, cysteine is added to a terminus of the partial peptide. When an inner sequence of the protein is selected, the N-terminus of the peptide is subjected to acetylation, and the C-terminus to amidation, if necessary.

The partial peptide can be synthesized by a general liquid phase peptide synthesis method or solid phase peptide synthesis method, optionally combined methods thereof or modified methods thereof [cf. *The Peptides, Analysis, Synthesis, Biology,* vol. 1, edited by Erhard Gross and Johannes Meinhofer, Academic Press, 1979, vol. 2, 1980, vol. 3, 1981; *Foundation and Experimentation of Peptide Synthesis*, Nobuo Izumiya *et al*., Maruzen, 1985; *Development of Medicament*, Second Series, vol. 14, *Peptide Synthesis,* edited by Haruaki Yajima, Hirokawa Shoten, 1991; *International Journal of Peptide Protein Research,* 35, 161 (1990)].

In addition, the partial peptide can also be synthesized using an automatic peptide synthesizer. Synthesis of a peptide by a peptide synthesizer can be carried out by a commercially available peptide synthesizer such as a peptide synthesizer manufactured by Shimadzu, a peptide synthesizer manufactured by Applied Biosystems, Inc., USA (hereinafter referred to as "ABI") or a peptide synthesizer manufactured by Advanced Chem Tech Inc., USA (hereinafter referred to as "ACT"), using appropriately side chain-protected Nα-Fmoc-amino acids or Nα-Boc-amino acids and the like and in accordance with respective synthesis program.

Also, the protected amino acids to be used as the material and the carrier resin can be purchased from ABI, Shimadzu, Kokusan Kagaku, Nova Biochem, Watanabe Kagaku, ACT, Peptide Laboratory and the like. Furthermore, protected amino acids, protected organic acids and protected organic amines to be used as the material of partial peptide can be synthesized by reported synthesis methods [cf. *The Peptides*, *Analysis, Synthesis, Biology*, vol. 1, edited by Erhard Gross and Johannes Meinhofer, Academic Press, 1979, vol. 2, 1980, vol. 3, 1981; *Foundation and Experimentation of Peptide Synthesis,* Nobuo Izumiya et al., Maruzen, 1985; *Development of Medicament*, *Second Series,* vol. 14, Peptide Synthesis, edited by Haruaki Yajima, Hirokawa Shoten, 1991; *International Journal of Peptide Protein Research,* 35, 161 (1990)] or in accordance therewith.

### (2) Immunization of animal and preparation of antibody-producing cell

Immunization is carried out by using the thus obtained protein as the antigen-The immunization is carried out by administering the antigen to the animal intravenously or intraperitonealy as it is. It is preferable that the antigen is administered by binding with a carrier protein having high antigenicity or together with an appropriate adjuvant.

The carrier protein includes *Macroschisma* hemocyanin, keyhole limpet hemocyanin, bovine serum albumin, bovine thyroglobulin and the like. The adjuvant includes complete Freund's adjuvant, aluminum hydroxide gel, pertussis vaccine and the like.

The animal to be immunized includes a non-human animal such as rabbit, goat, mouse, rat, hamster and the like.

The administration of the antigen is carried out three to ten times per week or 2 weeks after the first administration. A dosage of the antigen is preferably 50 to 100 µg per animal. After the administration, blood is collected from the fundus of the eye or tail vein of the immunized animal and the reactivity of the serum with the antigen is confirmed by enzyme immunoassay [*Enzyme-linked Immunosorbent Assay (ELISA)*, published by Igaku Shoin (1976)]. Then, a non-human animal showing a sufficient antibody titer in the sera is used as the supply source of serum or antibody-producing cells.

A monoclonal antibody can be prepared by fusing the antibody-producing cell and a myeloma cell derived from a non-human mammal to prepare a hybridoma, followed by culturing of the hybridoma, or administering the hybridoma into an animal to transform the cell into ascites tumor, and isolating and purifying the culture liquid or ascites. The antibody-producing cell is collected from spleen cells, lymph nodes or peripheral blood of the non-human mammal into which the antigen is administered.

### (3) Preparation of myeloma cell

Any myeloma cell can be used, so long as it proliferates *in vitro.* Examples include established cell lines obtained from mouse such as 8-azaguanine-resistant mouse (BALB/c) myeloma cell line P3-X63Ag8-U1 (P3-U1) [*Europ*. *J*. *Immunol*, 6, 511 (1976)], SP2/0-Ag14 (SP-2) [*Nature*, 276, 269 (1978)), P3-X63-Ag8653 (653) [*J*. *Immunol*., 123, 1548 (1979)], P3-X63-Ag8 (X63) [*Nature*, 256, 495 (1975)] and the like. These cell lines are cultured and subcultured according to the known method [*Antibodies - A Laboratory Manual*, Cold Spring Harbor Laboratory, Chapter 8 (1988), hereinafter referred to as "*Antibodies*"] and 2×10⁷ cells or more are secured until cell fusion.

### (4) Cell fusion and selection of monoclonal antibody

The above-obtained antibody-producing cells and myeloma cells are washed, a cell aggregating medium such as polyethylene glycol-1000 (PEG-1000) or the like, was added thereto to fuse the cells, and the cells are suspended in the medium. For washing the cells, MEM medium, PBS (1.83 g of disodium hydrogen phosphate, 0.21 g of potassium dihydrogen phosphate, 7.65 g of sodium chloride, 1 liter of distilled water, pH 7.2) or the like can be used. Also, in order to obtain the target fused cells selectively, HAT medium [normal medium (a medium prepared by adding glutamine (1.5 mM), 2-mercaptoethanol (5×10⁻⁵ M), gentamicin (10 µg/ml) and fetal calf serum (FCS) (10%, manufactured by CSL) to RPMI-1640 medium) further supplemented with hypoxanthine (10⁻⁴ M), thymidine (1.5×10⁻⁵ M) and aminopterin (4×10⁻⁷ M)], can be used as the medium for suspending the fused cells.

After the culturing, a portion of the culture supernatant is sampled and a sample which reacts with an antigen protein but does not react to a non-antigen protein is selected by enzyme immunoassay. Thereafter, cloning is carried out by a limiting dilution method, and a hybridoma which shows a stably high antibody titer is selected as the monoclonal antibody-producing hybridoma.

### Enzyme Immunoassay:

An antigen protein or an antigen-expressing cell is coated on a 96-well ELISA plate. A reaction is carried out using a hybridoma culture supernatant or a purified antibody obtained in the above method as a first antibody. After the reaction of the first antibody, the plate is washed and a second antibody is added thereto. The second antibody is obtained by labeling an antibody which can recognize immunoglobulin of the first antibody with biotin, an enzyme, a chemiluminescent substance, a radioactive compound or the like. Specifically, when a mouse is used for the production of the hybridoma, an antibody which can recognize mouse immunoglobulin is used as a second antibody. After the reaction, a reaction suitable for the substance used for labeling the second antibody is carried out to select a hybridoma producing a monoclonal antibody which specifically reacts with the antigen.

The hybridoma of the present invention includes hybridomas KM2895, KM2896, KM2897 and KM2904. Hybridomas KM2895 and KM2904 have been deposited on May 24, 2001, as FERM BP-7601 and FERM BP-7602, respectively, in International Patent Organism Depositary, National Institute of Advanced Industrial Science and Technology (AIST Tsukuba Central 6, 1-1, Higashi 1-Chome Tsukuba-shi, Ibaraki-ken, Japan).

### (5) Preparation of monoclonal antibody

The monoclonal antibody can be prepared by separation and purification from a culture obtained by culturing hybridoma cells or an ascitic fluid obtained by intraperitoneally administering monoclonal antibody-producing hybridoma cells into a 8- to 10-weeks-old mouse or nude mouse treated with pristane (0.5 ml of 2,6,10,14-tetramethylpentadecane (pristane) is intraperitoneally administered, followed by feeding for 2 weeks) and causing ascites tumor.

The method for separating and purifying the monoclonal antibody includes centrifugation, centrifugation, salting out with 40 to 50% saturated ammonium sulfate, caprylic acid precipitation, chromatography using a DEAE-Sepharose column, an anion exchange column, a protein A (or G) column or a gel filtration column, which may be used alone or in combination. According to the method, an IgG or IgM fraction can be recovered to obtain a purified monoclonal antibody.

The subclass of the purified monoclonal antibody can be determined using a monoclonal antibody typing kit or the like. The amount of the protein can be determined by the Lowry method or by absorbance at 280 nm.

The subclass of an antibody means isotypes within the class such as IgG1, IgG2a, IgG2b and IgG3 in the case of mouse, and IgG1, IgG2, IgG3 and IgG4 in the case of human. The mouse IgG1 and IgG2a and human IgG1 types have complement-dependent cytotoxic activity (hereinafter referred to as "CDC activity") and antibody-dependent cell-mediated cytotoxic activity (hereinafter referred to as "ADCC activity") so that they are useful in applying to medical treatments.

### 2. Preparation method of humanized antibody (I)

### Preparation method of anti-MT4-MMP catalytic domain humanized antibody:

### (1) Construction of vector for humanized antibody expression

A vector for humanized antibody expression required for production of a humanized antibody from an antibody derived from a non-human animal is constructed. The vector for humanized antibody expression is an expression vector for animal cell into which genes encoding, CH and CL, C regions of a human antibody have been inserted, and is constructed by cloning each of CH and CL of a human antibody into an expression vector for animal cell.

As the C region of a human antibody, C region of an optional human antibody, Cγ1 and Cγ4 in the case of human antibody H chains, and Cκ in the case of human antibody L chain, and the like can be used. As the gene encoding the C region of a human antibody, a chromosomal DNA comprising an exon and an intron or cDNA can be used. As the expression vector for animal cell, any expression vector can be used, so long as the C region of a human antibody can be inserted thereinto and expressed therein.

Examples include pAGE107 [*Cytotechnology*, 3, 133 (1990)], pAGE103 [*J*. *Biochem.,* 101, 1307 (1987)], pHSG274 [*Gene,* 27, 223 (1984)], pKCR [*Proc. Natl. Acad*. *Sci. USA,* 78, 1527 (1981)], pSGIβd2-4 [*Cytotechnology*, 4, 173 (1990)], and the like. A promoter and enhancer used for an expression vector for animal cell includes SV40 early promoter and enhancer [*J*. *Biochem.,* 101, 1307 (1987)], LTR promoter and enhancer of Moloney mouse leukemia virus [*Biochem. Biophys. Res. Comun.,* 149, 960 (1987)], promoter [*Cell,* 41, 479 (1985)] and enhancer [*Cell,* 33, 717 (1983)] of immunoglobulin H chain, and the like.

The vector for humanized antibody expression may be either of a type in which a gene encoding an antibody H chain and a gene encoding an antibody L chain exist on separate vectors or of a type in which both genes exist on the same vector (tandem type). In respect of easiness of construction of a humanized antibody expression vector, easiness of introduction into animal cells, and balance between the expression amounts of antibody H and L chains in animal cells, a tandem type of the humanized antibody expression vector is more preferred [*J*. *Immunol*. *Methods,* 167, 271 (1994)].

### (2) Preparation of cDNA encoding VH and VL of antibody derived from non-human animal

cDNAs encoding VH and VL of an antibody derived from an non-human animal such as a mouse anti-MT4-MMP catalytic domain monoclonal antibody are obtained as follows.

mRNA is extracted from a cell which produces an anti-MT4-MMP catalytic domain monoclonal antibody, for example, a hybridoma which produces an anti-MT4-MMP catalytic domain monoclonal antibody, to synthesize cDNA. The synthesized cDNA is inserted into a vector such as a phage, a plasmid or the like to prepare a cDNA library. Each of a recombinant phage or recombinant plasmid containing cDNA encoding VH and a recombinant phage or recombinant plasmid containing cDNA encoding VL is isolated from the library using a part of the C region or V region of a non-human antibody such as a mouse antibody as the probe. The full nucleotide sequences of the H chain V region and L chain V region of the mouse antibody of interest on the recombinant phage or recombinant plasmid are determined, and the full amino acid sequences of VH and VL are deduced from the nucleotide sequences.

### (3) Construction of human chimeric antibody expression vector

A human chimeric antibody expression vector can be constructed by inserting cDNAs encoding VH and VL of an antibody derived from a non-human animal into upstream of genes encoding CH and CL of a human antibody on the vector for humanized antibody expression as described in the above item 2(1). For example, the vector for human chimeric antibody expression can be constructed by preparing a recognition site of a restriction enzyme for cloning cDNAs encoding VH and VL of a non-human animal antibody in upstream of genes encoding CH and CL of a human antibody on the vector for chimeric antibody expression in advance, and inserting cDNA encoding an antibody derived from a non-human animal into the cloning site via a synthetic DNA described below. The synthetic DNA comprises a nucleotide sequence at the 3'-terminal of a V region of an antibody derived from a non-human animal and a nucleotide sequence at the 5'-terminal of an C region of a human antibody and can be produced by using a DNA synthesizer so as to have a recognition site at both ends.

### (4) Identification of CDR sequence of antibody derived from non-human animal

VH and VL constructing an antigen-binding site of an antibody comprise four framework regions (hereinafter referred to as "FR region") in which sequences are relatively preserved and three complementarity determining regions (CDR) for linking the FR region in which change of sequences are abundant [*Sequence of Proteins of Immunological Interest,* US Dept. Health and Human Services (1991); hereinafter referred to as *"Sequence of Proteins of Immunological Interest".* Each of the CDR amino acid sequences (CDR sequences) can be identifying by comparison with the amino acid sequence of the V region of a known antibody *(Sequences of Proteins of Immunological Interest).*

### (5) Construction of cDNA encoding V region of human CDR-grafted antibody

cDNAs encoding VH and VL of a human CDR-grafted antibody can be obtained as follows.

First, amino acid sequences of FRs in each of VH and VL of a human antibody to which amino acid sequences of desired CDRs in V region of an antibody derived from a non-human animal antibody are grafted are selected. Any amino acid sequences of FRs in V region of a human antibody can be used, so long as they are amino acid sequences of FRs in V region derived from human.

Examples include amino acid sequences of FRs in V region of human antibodies registered in database such as Protein Data Bank, and amino acid sequences common to subgroups of FRs in V regions of human antibodies (*Sequences of Proteins of Immunological Interest*). In order to provide a human CDR-grafted antibody having sufficient activity, the amino acid sequence preferably has high homology, preferably 65% or more, with the amino acid sequence in V region of an antibody of interest derived from a non-human animal. Then, DNA sequences encoding amino acid sequences of CDRs in V region of the antibody derived from a non-human animal are grafted to DNA sequences encoding the selected amino acid sequences of FRs of V region in a human antibody to design a DNA sequence encoding an amino acid sequence of each of VH and VL. In order to obtain a DNA sequence designed for constructing a CDR-grafted antibody V region gene, several synthetic DNAs are designed for each chain so as to cover all DNA sequences, and polymerase chain reaction (hereinafter referred to as "PCR") is carried out by using them. Preferably, six synthetic DNAs are designed for each chain based on reaction efficiency of the PCR and the length of the DNA which can be synthesized. After the reaction, amplified fragments are subcloned to an appropriate vector, and its nucleotide sequence is determined to obtain a plasmid comprising cDNA encoding an amino acid sequence of V region of each chain of the human CDR-grafted antibody of interest. Furthermore, all sense and anti-sense sequences are synthesized by using synthetic DNAs having a length of about 100 nucleotides, followed by annealing and ligating in order to construct cDNAs encoding an amino acid sequence of V region of each chain of the desired human CDR-grafted antibody.

### (6) Modification of amino acid sequence of V region of human CDR-grafted antibody

It is known that when a human CDR-grafted antibody is prepared by simply grafting CDRs in V region of an antibody derived from a non-human animal into FRs of V region of a human antibody, its antigen-binding activity is lower than that of the original antibody derived from a non-human animal [*BIO*/*TECHNOLOGY*, 9, 266 (1991)]. Accordingly, among the amino acid sequences of FRs in V region of a human antibody, an amino acid residue which directly relates to binding to an antigen, an amino acid residue which interacts with an amino acid residue in CDR or an amino acid residue which has a possibility of, for example, maintaining the three-dimensional structure of an antibody is modified with an amino acid residue which is found in the original non-human animal antibody to thereby increase the antigen binding activity. In order to efficiently identify these amino acid residues, construction and analysis of the three-dimensional structure of an antibody are carried out by X-ray crystallography, computer-modeling or the like. However, no method for preparing a human CDR-grafted antibody which can be applied to any antibodies has been established yet, so that various attempts must be currently necessary depending on each antibody.

The amino acid sequence of FRs in V region of a selected human antibody can be modified by using various primers for introducing mutation according to PCR as described in the above item 2(5). The amplified fragments after the PCR are subcloned into an appropriate vector, and then the nucleotide sequence is determined to obtain a vector comprising cDNA into which the mutation of interest is introduced (hereinafter referred to as "amino acid-modified vector").

Also, in modification of an amino acid sequence in a narrow range, a PCR mutation introducing method can be carried out by using primers for introducing mutation consisting of 20 to 35 nucleotides. Specifically, a sense mutation primer consisting of 20 to 35 nucleotides containing a DNA sequence encoding amino acid residues after the modification and its antisense mutation primer are synthesized, and two-stage PCR is carried out by using a plasmid containing cDNA encoding the amino acid sequence of the V region to be modified as the template. Final amplified fragments are subcloned into an appropriate vector, and then the amino acid sequence is determined to obtain an amino acid sequence-modified vector containing cDNA into which the mutation of interest is introduced.

### (7) Construction of human CDR-grafted antibody expression vector

A human CDR-grafted antibody expression vector can be constructed by introducing cDNA encoding VH and VL of the human CDR-grafted antibody obtained in the above items 2(5) and 2(6) into upstream of the gene encoding CH and CL of the human antibody in the vector for humanized antibody expression as described in the above item 2(1). For example, when recognition sites for an appropriate restriction enzymes are introduced to terminals of synthetic DNA of the 5'-terminal and 3'-terminal in the PCR for constructing cDNA encoding the amino acid sequences of VH and VL of the human CDR-grafted antibody, they can be introduced into upstream of the gene encoding the C region of a desired human antibody for expression in an appropriate form.

### (8) Transient expression and activity evaluation of humanized antibody

In order to efficiently evaluate the activities of various humanized antibodies, the human chimeric antibody expression vector described in the above item 2(3), the human CDR-grafted antibody expression vector in the above item 2(7) or a modified vector thereof is introduced into COS-7 cell (ATCCC RL1651) for carrying out transient expression of a humanized antibody [*Methods in Nucleic Acids Res.,* CRC Press, p. 283 (1991)], and its activity can be measured.

The method for introducing the expression vector into COS-7 cell includes a DEAE-dextran method [*Methods in Nucleic Acids Res.,* CRC Press, p. 283 (1991)], a lipofection method [*Proc*. *Natl*. *Acad*. *Sci*., *USA*, 84, 7413 (1987)], and the like.

After introduction of the vector, the activity of the humanized antibody in the culture supernatant can be determined by enzyme-linked immunosorbent assay (ELISA), and the like.

### (9) Stable expression and activity evaluation of humanized antibody

A transformant which produces a humanized antibody stably can be obtained by introducing the human chimeric antibody expression vector described in the above item 2(3) and the human CDR-grafted antibody expression vector described in the above item 2(7) into an appropriate host cell.

The method for introducing the expression vector into a host cell includes electroporation [Japanese Published Unexamined Patent Application No. 257891/90, *Cytotechnology*, 3, 133 (1990)] and the like.

Any cell can be used as the host cell into which the humanized antibody expression vector is to be introduced, so long as it can express a humanized antibody. Examples include mouse SP2/0-Ag14 cell (ATCC CRL1581), mouse P3X63-Ag8.653 cell (ATCC CRL1580), CHO cell in which a dihydrofolate reductase gene (hereinafter referred to as "DHFR gene") is detective [*Proc*. *Natl*. *Acad*. *Sci*. *U*.*S*.*A*., 77, 4216 (1980)], rat YB2/3HL.P2.G11.16Ag.20 cell (ATCC CRL1662, hereinafter referred to as "YB2/0 cell"), and the like.

After introduction of the expression vector, a transformant which expresses a humanized antibody stably is selected in accordance with the method disclosed in Japanese Published Unexamined Patent Application No. 257891/90 by using RPIM1640 medium containing G418 and FCS. The resulting transformant is cultured in a medium to produce and accumulate a humanized antibody in the culture. The activity of the humanized antibody in the culture is measured by the method described in the above item 1 (4) and the like. Also, in the transformant, the expression amount of the humanized antibody can be increased by using DHFR gene amplification system or the like according to the method disclosed in Japanese Published Unexamined Patent Application No. 257891/90.

The humanized antibody can be purified from the culture supernatant of the transformant by using a protein A column (*Antibodies,* Chapter 8). Any other conventional methods for protein purification can be used. For example, the humanized antibody can be purified by a combination of gel filtration, ion-exchange chromatography, ultrafiltration and the like. The molecular weight of the H chain or the L chain of the purified humanized antibody or the antibody molecule as a whole is determined by polyacrylamide gel electrophoresis (hereinafter referred to as "SDS-PAGE") [*Nature*, 227, 680 (1970)], Western blotting (*Antibodies,* Chapter 12), or the like.

The reactivity of the purified humanized antibody and the binding activity of the humanized antibody to an MT4-MMP catalytic domain can be measured by the method described in the above item 1(4) and the like.

### 3. Preparation method of recombinant antibody (II)

### (1) Preparation method of antibody fragments Fab, Fab' and F(ab')₂

An antibody fragment can be prepared by treating the above-described antibody with an enzyme. The enzyme includes papain, trypsin and the like.

Also, DNA encoding Fab, Fab' or F(ab')₂ fragment of the anti-MT4-MMP catalytic domain antibody is inserted into an expression vector for animal cell, and the vector is introduced into an animal cell for expression to prepare Fab, Fab' or F(ab')₂.

The antibody fragment can be purified by a combination of gel filtration, ion-exchange chromatography, ultrafiltration and the like. The molecular weight of the purified Fab, Fab' or F(ab')₂ is determined by polyacrylamide gel electrophoresis (SDS-PAGE) [*Nature*, 227, 680 (1970)], Western blotting (*Antibodies,* Chapter 12), or the like.

The reactivity of the purified Fab, Fab' or F(ab')₂ and the binding activity of the Fab, Fab' or F(ab')₂ to an MT4-MMP catalytic domain can be measured by the method described in the above item 1 (4) and the like.

### (2) Preparation method of anti-MT4-MMP catalytic domain single chain antibody

cDNAs encoding VH and VL of the antibody derived from a non-human animal or the human CDR-grafted antibody described in the above items 2(2), 2(5) and 2(6) are introduced into a vector for single chain antibody expression to construct a single chain antibody expression vector of an antibody derived from a non-human animal or a single chain antibody expression vector of a human CDR-grafted antibody. Any of the vector for single chain antibody expression can be used, so long as cDNAs encoding VH and VL of the antibody derived from a non-human animal or the human CDR-grafted antibody can be inserted for expression. Examples includes pAGE107 [*Cytotechnology*, 3, 133 (1990)], pAGE103 [*J*. *Biochemistry*, 101, 1307 (1987)], pHSG274 [*Gene,* 27, 223 (1984)], pKCR [*Proc*. *Natl*. *Acad*. *Sci*. *U*.*S*.*A*., 78, 1527 (1981)], pSG1βd2-4 [*Cytotechnology*, 4, 173 (1990)] and the like. As a host for expression of the single chain antibody, an appropriate host can be selected from *Escherichia coli*, yeast, animal cell and the like, and in this case, a vector for expression suitable for the host can be selected.

Furthermore, when a cDNA encoding an appropriate signal peptide is inserted into the vector for expression, the single chain antibody can be secreted extracellularly, be transported to the periplasmic space or be remained intracellularly.

cDNA encoding a single chain antibody comprising VH-P-VL or VL-P-VH (P is a peptide linker) is inserted into downstream of an appropriate vector and a signal peptide of the selected vector for expression to construct a single chain antibody expression vector into which cDNA encoding the single chain antibody of interest is inserted.

cDNA encoding the single chain antibody can be obtained by linking cDNA encoding VH and cDNA encoding VL using a synthetic DNA encoding a peptide linker having recognition sites of appropriate restriction enzymes at both terminals. It is important that the linker peptide is optimized so as not to inhibit binding of VH and VL to an antigen as a result of addition of the linker peptide. For example, linker peptide described by Pantoliano *et al*. [*Biochemistry*, 30, 10117 (1991)] or the modified linker peptide thereof can be used.

### (3) Preparation method of anti-MT4-MMP catalytic domain disulfide stabilized antibody

A disulfide stabilized antibody can be prepared by modifying a DNA sequence corresponding to one amino acid residue at an appropriate position of each of cDNAs encoding VH and VL of an antibody derived from a non-human animal or of cDNAs encoding VH and VL of a human CDR-grafted antibody with a DNA sequence corresponding to a cysteine residue, followed by expression and purification to form a disulfide bond. The modification of the amino acid residue with the cysteine residue can be carried out according to the mutation introducing method using PCR as described in the above item 2(5). The resulting cDNAs encoding the modified VH and the modified VL are expressed in an appropriate vector for expression to construct a disulfide stabilized antibody H chain expression vector and a disulfide stabilized antibody L chain expression vector. Any of the vector for disulfide stabilized antibody expression can be used, so long as cDNAs encoding the modified VH and the modified VL can be inserted for expression. Examples includes pAGE107 [*Cytotechnology*, 3, 133 (1990)], pAGE103 [*J*. *Biochemistry*, 101, 1307 (1987)], pHSG274 [*Gene*, 27, 223 (1984)], pKCR [*Proc*. *Natl*. *Acad*. *Sci*. *U*.*S*.*A*., 78, 1527 (1981)], pSG1βd2-4 [*Cytotechnology*, 4, 173 (1990)] and the like. As a host for expression of the disulfide stabilized antibody L chain expression vector and disulfide stabilized antibody H chain expression vector for forming the disulfide stabilized antibody, an appropriate host can be selected from *Escherichia coli,* yeast, animal cell and the like, and in this case, a vector for expression suitable for the host can be selected.

Furthermore, when a cDNA encoding an appropriate signal peptide is inserted into the vector for expression, the disulfide stabilized antibody can be secreted extracellularly, be transported to the periplasmic space or be remained intracellularly.

### (4) Expression and activity evaluation of various antibodies

A transformant which produces the antibody fragment, single chain antibody, disulfide stabilized antibody H chain or disulfide stabilized antibody L chain of interest can be obtained by introducing into a host cell the antibody fragment expression vector, single chain antibody expression vector, disulfide stabilized antibody H chain expression vector or disulfide stabilized antibody L chain expression vector constructed in the above items 3(1) to 3(3) by electroporation [Japanese Published Unexamined Patent Application No. 257891/90, *Cytotechnology*, 3, 133 (1990)] and the like. After the introduction of the expression vector, expression of the antibody fragment, single chain antibody, disulfide stabilized antibody H chain or disulfide stabilized antibody L chain contained in the culture supernatant or the like can be confirmed by the method described in the above item 1(4) or the like.

Recovery and purification of the single chain antibody, the disulfide stabilized antibody H chain or the disulfide stabilized antibody L chain can be accomplished by combining known techniques. For example, when the antibody fragment, the single chain antibody, the disulfide stabilized antibody H chain or the disulfide stabilized antibody L chain is secreted into a medium, the recovery and purification can be accomplished by concentration using ultrafiltration, followed by various chromatography or gel filtration. Furthermore, when it is transported to the periplasmic space of the host cell, they can be accomplished by applying osmotic shock to the host cell, followed by concentration using ultrafiltration and subsequent various chromatography or gel filtration. When the antibody fragment, the single chain antibody, the disulfide stabilized antibody H chain or the disulfide stabilized antibody L chain is insoluble and present in the form of an inclusion body, they can be accomplished by repeating centrifugation and washing for solubilizing the cells and isolating the inclusion bodies, followed by solubilization with, for example, guanidine - hydrochloric acid, and subsequent various chromatography or gel filtration.

The activity of the purified single chain antibody can be measured by the method described in the above item 1 (4). The purified disulfide stabilized antibody H chain and disulfide stabilized antibody L chain are mixed and a disulfide bond is formed by operation leading to a structure having an activity [Refolding operation, *Molecular Immunology*, 32, 249 (1995)], and the disulfide stabilized antibody having an activity can be purified by antigen affinity chromatography, ion exchange chromatography or gel filtration. The activity of the disulfide stabilized antibody can be measured by the method described in the above item 1(4) or the like.

### 4. Preparation method of fusion antibody

A fusion antibody prepared by linking the antibody or antibody fragment used in the present invention with a radioisotope, a protein, a low molecular weight agent or the like chemically or genetically can also be used as an antibody derivative.

A fusion antibody in which an antibody and a toxin protein are linked chemically can be prepared in accordance with the method described in literatures [*Anticancer Research*, 11, 2003 (1991); *Nature Medicine,* 3, 350 (1996)].

A fusion antibody in which an antibody and a protein such as toxin, cytokine or the like are linked genetically can be prepared in accordance with the method described in literatures [*Proc*. *Natl*. *Acad Science, USA*, 93, 974 (1996); *Proc. Natl*. *Acad Science, USA,* 93, 7826 (1996)].

A fusion antibody in which an antibody and a low molecular weight agent are linked chemically can be prepared in accordance with methods described in literatures [*Science,* 261, 212 (1993)]. A fusion antibody in which an antibody and a radioisotope are linked chemically can be prepared in accordance with the method described in literatures [*Antibody Immunoconjugates and Radiopharmaceuticals*, 3, 60 (1990); *Anticancer Research,* 11, 2003 (1991)].

Since these derivatives can accumulate a radioisotope, a protein (a cytokine, a toxin, an enzyme or the like), a low molecular weight agent or the like into the periphery of a target tissue according to the specificity of the antibody molecule, a diagnosis or treatment which is more effective and has less side effects can be expected from them.

### 5. Application method of antibody (I)

The above anti-MT4-MMP catalytic domain antibody, the antibody fragment or the fusion antibody thereof with other molecule binds to an MT4-MMP catalytic domain and destroys cells expressing MT4-MMP on the cell surface via effector activity of the antibody such as antibody-dependent cell-mediated cytotoxic activity (ADCC activity) and complement-dependent cytotoxic activity (CDC activity). Thus, they are useful in treating diseases relating to MT4-MMP.

The diseases relating to MT4-MMP include osteoarthritis, rheumatoid arthritis, asthma, autoimmune diseases, atopic dermatitis, psoriasis, contact dermatitis, alopecia, ischemic heart disease, immune reaction accompanied by organ transplantation, hepatitis, glomerulonephritis, pancreatitis, arteriosclerosis, leukemia, malignant tumor, injury, corneal tumor, tissue injury, inflammation accompanied by infiltration of leukocyte and the like.

The medicament comprising the antibody of the present invention can be administered as a therapeutic agent alone, but generally, it is preferred to provide it as a pharmaceutical formulation produced by an appropriate method well known in the technical field of pharmaceutical, by mixing it with at least one pharmaceutically acceptable carrier.

It is preferable to select a route of administration which is most effective in treatment. Examples include oral administration and parenteral administration, such as buccal, tracheal, rectal, subcutaneous, intramuscular and intravenous. In an antibody preparation, intravenous administration is preferred.

The dosage form includes sprays, capsules, tablets, granules, syrups, emulsions, suppositories, injections, ointments, tapes and the like.

The pharmaceutical preparation suitable for oral administration include emulsions, syrups, capsules, tablets, powders, granules and the like.

Liquid preparations such as emulsions and syrups can be produced by using, as additives, water; saccharides such as sucrose, sorbitol and fructose; glycols such as polyethylene glycol and propylene glycol; oils such as sesame oil, olive oil and soybean oil; antiseptics such as p-hydroxybenzoic acid esters; flavors such as strawberry flavor and peppermint; and the like.

Capsules, tablets, powders, granules and the like can be produced by using, as additive, excipients such as lactose, glucose, sucrose and mannitol; disintegrating agents such as starch and sodium alginate; lubricants such as magnesium stearate and talc; binders such as polyvinyl alcohol, hydroxypropylcellulose and gelatin; surfactants such as fatty acid ester; plasticizers such as glycerine; and the like.

The pharmaceutical preparation suitable for parenteral administration includes injections, suppositories, sprays and the like.

Injections may be prepared by using a carrier such as a salt solution, a glucose solution or a mixture of both thereof.

Suppositories may be prepared by using a carrier such as cacao butter, hydrogenated fat or carboxylic acid.

Also, sprays may be prepared by using the antibody composition as such or using a carrier and the like which does not stimulate the buccal or airway mucous membrane of the patient and can facilitate absorption of the antibody composition by dispersing it as fine particles.

The carrier includes lactose, glycerol and the like. Depending on the properties of the antibody composition and the carrier, it is possible to produce pharmaceutical preparations such as aerosols and dry powders. In addition, the components exemplified as additives for oral preparations can also be added to the parenteral preparations.

Although the clinical dose or the frequency of administration varies depending on the objective therapeutic effect, administration method, treating period, age, body weight and the like, it is usually 10 µg/kg to 8 mg/kg for adult patients per day.

Also, the method for examining antitumor effect of the antibody used in the present invention against various tumor cells include *in vitro* tests such as CDC activity measuring method, ADCC activity measuring method, and *in vivo* tests such as antitumor experiments using a tumor system in an experimental animal such as a mouse.

CDC activity and ADCC activity measurements and antitumor experiments can be carried out in accordance with the methods described in literatures [*Cancer Immunology Immunotherapy,* 36, 373 (1993); *Cancer Research,* 54, 1511 (1994)] and the like.

### 6. Application method of antibody (II)

Also, the present invention relates to a method for immunologically detecting and determining an MT4-MMP catalytic domain or a microorganism, animal cell or insect cell expressing the domain extracellularly by using the monoclonal antibody of the present invention.

The method for immunologically detecting or determining a microorganism, animal cell or insect cell expressing the MT4-MMP catalytic domain extracellularly by using the monoclonal antibody of the present invention includes immunological assays such as fluorescent antibody technique, enzyme-linked immunosorbent assay (ELISA) and radioimmunoassay (RIA), immunohistochemical staining methods (ABC method, CSA method, *etc.*) such as tissue immunostaining and cell immunostaining, Western blotting, dot blotting, immunoprecipitation (*Monoclonal Antibody Experimentation Manual*, Kodansha Scientific, 1987; *A Sequel to Series Biochemistry Experimentation Course*, 5. Method for Studying Immunological Biochemistry, Tokyo Kagaku Dojin, 1986) and the like.

As the immunological assay, any known immunological assays can be used. As described above, examples include radioimmunoassay (RIA), enzyme immunoassay (EIA or ELISA), fluoroimmunoassay (FIA), luminescent immunoassay, physicochemical detection method (TIA, LAPIA or PCIA) and the like based on the difference in the labeling method, and the enzyme immunoassay is preferred.

As the label used in the enzyme immunoassay, any known (*Enzyme Immunoassay*, edited by Eiji Ishikawa *et al*., Igaku Shoin) enzyme label can be used. For example, an alkaline phosphatase label, a peroxidase label, a luciferase label and the like can be used.

As the label used in the luminescent immunoassay, any known [*Bioluminescence and Chemiluminescence,* edited by Kazuhiro Imai, Hirokawa Shoten; *Rinsho Kensa*, 42 (1998)] luminescent label can be used. For example, an acridinium ester label, a lophine label and the like can be used.

As the label used in the fluoroimmunoassay, any known (*Fluorescent Antibody Technique,* edited by Akira Kawaoi, Soft Science) fluorescent label can be used. For example, an FITC label, an RITC label and the like can be used.

The immunological assay is a method in which the amount of an antibody or the amount of an antigen is measured by using an antigen or antibody treated with the above various labels. The immunological assay of the present invention may be any method for carrying out detection or measurement of antigens. For example, a competitive method and a sandwich method [*Immunology Illustrated,* 5th edition, Nankodo] can be exemplified, and a sandwich method is preferred.

The sandwich method is a method in which a primary antibody is linked to a solid phase to trap an antigen to be measured and then allowed to react with a labeled secondary antibody. The antibodies used in the sandwich method may be either polyclonal antibodies or monoclonal antibodies, or antibody fragments such as the above Fab, Fab' and F(ab)₂ can be used. The combination of the two antibodies used in the sandwich method may be a combination of monoclonal antibodies or antibody fragments which recognize different epitopes or may be a combination of a polyclonal antibody with a monoclonal antibody or an antibody fragment. A combination of KM2895 with KM2904 which are monoclonal antibodies of the present invention is preferred.

The fluorescent antibody technique is a method in which the monoclonal antibody of the present invention is allowed to react with a microorganism, animal cell or insect cell expressing the MT4-MMP catalytic domain extracellularly and further allowed to react with an anti-mouse IgG antibody or binding fragment labeled with a fluorescent material such as fluorescent isothiocyanate (FITC), and then the fluorescence dye is measured by using a flow cytometer.

The enzyme-linked immunosorbent assay (ELISA) is a method in which the monoclonal antibody of the present invention is allowed to react with a microorganism, animal cell or insect cell expressing the MT4-MMP catalytic domain extracellularly and further allowed to react with an anti-mouse IgG antibody or binding fragment treated with an enzyme label such as peroxidase or biotin, and then the colored dye is measured by using a flow cytometer.

The radioimmunoassay (RIA) is a method in which the monoclonal antibody of the present invention is allowed to react with a microorganism, animal cell or insect cell expressing the MT4-MMP catalytic domain extracellularly and further allowed to react with an anti-mouse IgG antibody or binding fragment treated with a radioisotope label and then the isotope is measured by using a scintillation counter or the like.

The cell immunostaining or tissue immunostaining is a method in which the monoclonal antibody of the present invention is allowed to react with a microorganism, animal cell or insect cell expressing the MT4-MMP catalytic domain extracellularly and further allowed to react with an anti-mouse IgG antibody or binding fragment treated with a fluorescent material such as FITC or an enzyme label such as peroxidase or biotin and then the cell or tissue is observed under a microscope.

The Western blotting is a method in which a cell extract of a microorganism, animal cell or insect cell expressing the MT4-MMP catalytic domain extracellularly is fractionated by SDS-polyacrylamide gel electrophoresis (*Antibodies - A Laboratory Manual*, Cold Spring Harbor Laboratory, 1988), the gel is blotted on a PVDF membrane or nitrocellulose membrane, the membrane is allowed to react with the monoclonal antibody of the present invention and further allowed to react with an anti-mouse IgG antibody or binding fragment treated with a fluorescent material such as FITC or an enzyme label such as peroxidase or biotin, and then the result is confirmed.

The dot blotting is a method in which a cell extract of a microorganism, animal cell or insect cell expressing the MT4-MMP catalytic domain extracellularly is blotted on a nitrocellulose membrane, the membrane is allowed to react with the monoclonal antibody of the present invention and further allowed to react with an anti-mouse IgG antibody or binding fragment treated with a fluorescent material such as FITC or an enzyme label such as peroxidase or biotin, and then the result is confirmed.

The immunoprecipitation is a method in which a cell extract of a microorganism, animal cell or insect cell expressing the MT4-MMP catalytic domain extracellularly is allowed to react with the monoclonal antibody of the present invention and then an antigen-antibody complex is precipitated by adding a carrier having immunoglobulin-specific binding ability such as protein G-Sepharose.

The method for diagnosing diseases relating to MT4-MMP includes a method in which the MT4-MMP catalytic domain is immunologically detected or determined as described above by using various human tumor cultured cells or cells collected from patients by biopsy *etc.* and cell extracts prepared from the cells. Accordingly, the monoclonal antibody of the present invention can be used as a diagnostic agent for the above diseases relating to MT4-MMP.

### 7. Application method of antibody (III)

Furthermore, the anti-MT4-MMP catalytic domain monoclonal antibody of the present invention can be used for purifying MT4-MMP. Specifically, affinity chromatography is carried out by using the antibody of the present invention.

The anti-MT4-MMP catalytic domain monoclonal antibody is immobilized on the carrier to prepare an antibody column by using a carrier having immunoglobulin-specific binding ability such as protein G-Sepharose or using various coupling gels which directly binds to immunoglobulin via an amino group,.

As the sample, cell extracts of animal cells or insect cells expressing the MT4-MMP or cell extracts of various human tumor cultured cells or cells collected from patients by biopsy *etc.* can be used.

The above MT4-MMP sample is applied to the antibody column and then washed with 10 folds of the column volume of a phosphate buffer (pH 7.2) containing 0.5 M NaCl. Thereafter, the purified MT4-MMP is obtained by eluting it with a buffer under conditions for dissociate the antigen-antibody reaction (high pH, low pH, high salt concentration, surfactant, denaturing agent and the like). Also, it is necessary to carry out the elution under such conditions that enzyme activity of MT4-MMP is not inactivated.

The present invention is hereinafter explained in detail according to Examples, but the present invention is not limited to these Examples.

### BRIEF DESCRIPTION OF THE DRAWINGS

Fig. 1 is a graph showing results of the enzyme immunoassay examination of reactivity of the monoclonal antibody of the present invention obtained by using MT4-MMP catalytic domain as the antigen, against MT4-MMP catalytic domain or *Escherichia coli* protein.
Fig. 2 is a chart showing results of measurement of MT4-MMP protein distributing on the membrane of MT4-MMP gene-introduced COS-1 cell, detected by cell immunostaining using the monoclonal antibody of the present invention. COS-1 and MT5-MMP gene-introduced COS-1 were used as control cells. The abscissa and the ordinate show fluorescence intensity and the number of cells, respectively.
Fig. 3 is a graph showing results of the detection of MT4-MMP protein and MT5-MMP protein by a sandwich ELISA system using the monoclonal antibody of the present invention. "○" and "●" show reactivity for MT4-MMP and reactivity for MT5-MMP, respectively.
Fig. 4 shows results of the detection of MT4-MMP protein existing in sera of mouse collagen-induced arthritis (CIA) model by a sandwich ELISA system using the monoclonal antibody of the present invention. Amounts of MT4-MMP protein in sera. of normal rats and arthritis-induced rats are compared.

### BEST MODE FOR CARRYING OUT THE INVENTION

### Example 1

### Expression of human MT4-MMP catalytic domain protein:

### (1) Sequence information of human MT4-MMP gene

As the information of human MT4-MMP cDNA, a sequence registered with one of the gene sequence data bases, Genbank, (Accession No. X89576). Its nucleotide sequence is shown in SEQ ID NO:5. The nucleotide sequence encoding a human MT4-MMP catalytic domain corresponds to a nucleotide sequence of positions 481 to 987 in SEQ ID NO:5.

### (2) Design of primers used in amplification of human MT4-MMP catalytic domain

Unless otherwise indicated, gene engineering techniques were carried out by the methods described in *Molecular Cloning,* 2nd edition.

Nucleotide sequences of the primers used in the amplification of human MT4-MMP catalytic domain are shown in SEQ ID NOs:1 and 2.

Also, primers for nucleotide sequence confirmation were designed from sequences positioned in both sides of the cloning site of a cloning vector pCR-Blunt (manufactured by Invitrogen, Carlsbad, CA, USA). Nucleotide sequences of the primers for nucleotide sequence confirmation are shown in SEQ ID NOs:3 and 4.

### (3) Amplification of human MT4-MMP catalytic domain by PCR method

As the template of PCR, 1 µl of a plasmid hMT4Δt/pRSET B (WO00/18805) was used. Using PLATINUM pfx DNA Polymerase (manufactured by Life Technologies) as a heat resistant enzyme, PCR was carried out by using 50 ml in total of a reaction solution [1×pfx Amplification buffer, 1 mM MgSO₄, 300 mM of each of dNTP (dATP, dGTP, dCTP and dTTP), 0.3 mM of each of the primers MT4-MMPCDF1 and MT4-MMPCDR1 obtained in the above item (2), and 1.25 units of PLATINUM pfx DNA Polymerase]. Using Thermal Cycler PTC-200 (manufactured by MJ RESEARCH), the solution was heated at 94°C for 2 minutes, subjected to 25 cycles of a reaction at 94°C for 15 seconds, at 60 °C for 30 seconds and 68°C for 1 minute as one cycle, and then further heated at 68°C for 10 minutes. By agarose electrophoresis, 5 ml of the PCR reaction solution was analyzed to confirm that the expected DNA fragment of about 0.5 kb was amplified.

### (4) Purification of the amplified DNA fragment and its insertion into a vector

All of the remaining PCR reaction solution was separated by agarose electrophoresis to recover the DNA fragment of about 0.5 kb. The DNA fragment was purified by using QIAEX II gel extraction kit (manufactured by QUIAGEN) in accordance with the manufacture's instruction attached to the kit. As the vector DNA, pCR-Blunt attached to Zero Blunt PCR Cloning Kit (manufactured by Invitrogen) was used. About 50 ng of the purified PCR product was mixed with about 25 ng of pCR-Blunt, and the DNA solution was mixed with the same volume of TaKaRa ligation system ver. 2 (manufactured by Takara Shuzo) to carry out ligation at 16°C for 12 hours. Using the recombinant plasmid DNA obtained by the reaction, *E. coli* TOP10 [genotype: F⁻, mcrA Δ(mrr-hsdRMS-mcrBC) φ80 lacZΔM15 ΔlacX74 deoR recA1 araD139 Δ(ara-leu)7697 galU galK rps(Str^{R}) endA1 nupG] was transformed to obtain a plasmid pCR-BluntMT4MMPCD.

### (5) Confirmation of pCR-BluntMT4MMPCD nucleotide sequence

Confirmation of the nucleotide sequence of cDNA introduced into pCR-BluntMT4MMPCD was carried out by using ABI PRISM 377 DNA Sequencer (manufactured by PE Applied Biosystems). The sequencing reaction was carried out by using Big Dye Terminator Cycle Sequencing Ready Reaction (manufactured by PE Applied Biosystems) in accordance with the manufacture's instruction attached to the kit. Using the primers Fl and R1 obtained in the above item (2) as the primers for sequencing, nucleotide sequences of both chains were confirmed. GENETYX WIN ver. 3.2 (manufactured by Software) was used in the sequence data analysis.

When the cloned nucleotide sequence was confirmed, it coincided with a nucleotide sequence of positions 484 to 1005 of a known sequence (Genbank Accession No. X89576) shown in SEQ ID NO:5. This sequence corresponds to the human MT4-MMP catalytic domain and hinge region.

### (6) Recombination from pCR-Blunt to expression vector pET23a(+)

A DNA containing the MT4-MMP catalytic domain was cut out by digesting about 6 mg of the pCR-BluntMT4MMPCD at 37°C for 1 hour in 40 µl of a reaction solution comprising 10 mM Tris-HCl (pH 7.5), 10 mM MgCl₂, 100 mM NaCl, 0.01% bovine serum albumin (BSA), 1 mM dithiothreitol (DTT), 30 units of *Nde*I (manufactured by New England Biolabs) and 15 units of *Not*I (manufactured by Takara Shuzo). By separating the reaction solution by agarose electrophoresis, an *Nde*I-*Not*I DNA fragment of about 0.5 kb was recovered. The DNA fragment was purified by using QIAEX II gel extraction kit (manufactured by QIAGEN).

Digestion of 5 µg of pET23a(+) (manufactured by Novagen) was carried out at 37°C for 2 hours in 40 µl of a reaction solution comprising 10 mM Tris-HCl (pH 7.5), 10 mM MgCl₂, 100 mM NaCl, 0.01% BSA, 1 mM DTT, 30 units of *Nde*I (manufactured by New England Biolabs) and 15 units of *Not*I (manufactured by Takara Shuzo). By separating the reaction solution by agarose electrophoresis, a DNA fragment of about 3.7 kb was recovered. The DNA fragment was purified by using QIAEX II gel extraction kit (manufactured by QIAGEN).

About 50 ng of the above an *Nde*I*-Not*I fragment containing the above MT4-MMP catalytic domain was mixed with about 50 ng of the pET23a(+) fragment which had been treated with *Nde*I and *Not*I, and the DNA solution was mixed with the same volume of TaKaRa ligation system ver. 2 (manufactured by Takara Shuzo) to carry out the ligation at 16°C for 2 hours. Using the recombinant plasmid DNA obtained by the reaction, *E. coli* XL-1 Blue MRF' [genotype: Δ(mcrA)183 Δ(mcrCB-hsdSMR-mrr)173 endA1 supE44 thi-1 recA1 gyrA96 reLA1 lac [F proAB lacI^{q} ZΔM15 Tn10(Tet^{r}]] was transformed to obtain a plasmid pET23aMT4MMPCD. In the same manner, the *Nde*I-*Not*I fragment containing the MT4-MMP catalytic domain was inserted into pET24a(+) (manufactured by Novagen) to prepare pET24aMT4-MMPCD.

### (7) Introduction of pET23aMT4-MMPCD into E. coli BL21/DE3pLysS

*E*. *coli* BL21/DE3pLysS [genotype: F⁻ ompT hsdS_{B}(r_{B}⁻m_{B}⁻)gal dcm(DE3) pLysS] is a 1 phage, 1 DE3 lysogenic bacterium having T7 RNA polymerase gene in the downstream of lacUV5 promoter. When the BL21/DE3pLysS introduced with an expression plasmid is cultured and induction is carried out with isopropylthio-b-D-galactoside (IPTG), the T7 RNA polymerase is firstly expressed and then strong transcription of the gene of interest is started. Thus, pET23aMT4-MMPCD was introduced into BL21/DE3pLysS. To 20 µl of BL21/DE3pLysS competent cell (manufactured by Novagen), 1 µg of pET23aMT4MMPCD was added thereto, and the mixture was allowed to stand on ice for 10 minutes. After heat shock is applied at 42°C for 30 seconds, the mixture was placed on ice for 2 minutes, 80 µl of SOC (attached to the kit of competent cell) was added thereto, and then the mixture was allowed to stand at 37°C for 30 minutes. A whole amount thereof was inoculated onto Luria Bertani (LB) plate medium comprising 50 µg/ml ampicillin sodium salt (manufactured by Nacalai Tesque) and cultured at 37°C until colonies were grown to a diameter of about 1 mm.

### (8) Expression induction of human recombinant MT4-MMP catalytic domain

Culturing of the recombinant *E*. *coli* was carried out using LB liquid medium containing 100 µg/ml of ampicillin sodium salt (manufactured by Nacalai Tesque).

The recombinant *E*. *coli* was inoculated into 2 ml of the medium and then cultured on a constant temperature shaker BIO-SHAKER (model BR-40LF, manufactured by Taiteck) at 37°C and 200 revolution/minute (rpm) overnight. Onto 75 ml of the medium, 0.5 ml of the pre-cultured medium was inoculated to start the main culturing. After shaking culture at 37°C and 200 rpm for 2 hours, IPTG (manufactured by Nacalai Tesque) was added to give a final concentration of 1 mM, and the shaking culture was continued at 37°C and 200 rpm for 4 hours to induce expression of human MT4-MMP catalytic domain.

### (9) Purification of human recombinant MT4-MMP catalytic domain

The expression-induced cells using 75 ml of the medium were washed once with 50 ml of 50 mM Tris-HCl (pH 7.4). To the cells, 300 ml of 50 mM Tris-HCl (pH 7.4) containing 0.1% Triton X-100, 10 mM EDTA (pH 8.0), 10 µg/ml DNase I (manufactured by Sigma-Aldrich), 0.1 mM phenylmethylsulfonyl fluoride (PMSF) (manufactured by Sigma-Aldrich), 0.1 µg/ml leupeptin (manufactured by Peptide Laboratory) and 0.1 µg/ml pepstatin (manufactured by Peptide Laboratory) was added, followed by incubation at 37°C for 2 hours. After centrifugation, the thus recovered precipitate was washed 4 times with 50 mM Tris-HCl (pH 7.4) comprising 10 mM EDTA (pH 8.0) and the above protease inhibitor. The precipitate was dissolved by adding 2 ml of 50 mM Tris-HCl (pH 7.4) comprising 8 M urea and dispensed into 1.5 ml capacity tubes and then centrifuged at 15,000 rpm and at 4°C for 20 minutes to recover the supernatant. The supernatant was desalted by using a handy filtration column PD-10 (manufactured by Amersham Pharmacia Biotech) and stored at -20°C.

### (10) Measurement of enzyme activity

To 190 µl of an assay buffer comprising 10 µM of a fluorescent substrate MOCAc-Pro-Leu-Gly-Leu-A2pr(Dnp)-Ala-Arg-NH₂ (manufactured by Peptide Laboratory), 50 mM CaCl₂, 0.005% Brij 35 and 62.5 nM ZnCl₂, (final concentration: 0.8 µg/ml), 10 µl of a 16 µg/ml enzyme solution (diluted with 50 mM Tris-HCl (pH 7.4) containing 8 M urea) was added, followed by reaction for 30 to 240 minutes, and then the fluorescence intensity was measured. Determination of the amount of the enzyme was carried out by using Protein Assay reagent (manufactured by Bio-Rad) and using BSA as a standard. Also, using MOCAc-Pro-Leu-Gly (manufactured by Peptide Laboratory) as a standard fluorescent substrate, a relationship between a fluorescence value and a substrate degradation was calculated (fluorescence value = substrate degradation × 6943.4 + 116.48). The measurement was carried out by using ARVO (manufactured by Beltold Japan) under conditions of Ex. 320, Em. 393, Energy 2000 and 1 second.

By carrying out expression induction using the pET system, 2.5 ml of a 160 µg/ml enzyme solution was obtained from 75 ml of the cultured cells. When the enzyme activity was measured, the MT4-MMP activity was detected and the substrate degradation rate was 1.2 µmol/mg protein/hour.

The resulting product was generally dissolved in 8 M urea (manufactured by Nacalai Tesque) and stored at 4°C, but when used, the product was diluted 20-fold or more with the above buffer, and the mixture was allowed to stand at 25°C for 90 minutes for refolding, followed by optional dilution.

### Example 2

### Preparation of monoclonal antibody which specifically binds to anti-MT4-MMP catalytic domain:

### (1) Preparation of immunogen

The MT4-MMP catalytic domain obtained in Example 1 was added to a solution comprising 50 mM Tris-HCl (pH 7.4), 50 mM CaCl₂, 500 nM ZnCl₂ and 0.005% Brij 35, and the mixture was allowed to stand at room temperature for 1 hour for refolding and then used as the immunogen.

### (2) Immunization of animal and preparation of antibody-producing cell

To 5-week-old mice (Balb/c) or week-old rats (SD), 50 µg of the MT4-MMP catalytic domain prepared in Example 2(1) was administered, together with 2 mg of aluminum gel and 1×10⁹ cells of pertussis vaccine (manufactured by Chiba Serum Institute), and 2 weeks thereafter, administration of 100 µg of the MT4-MMP catalytic domain was started once a week for a total of 4 times. Blood samples were collected from the venous plexus of the fundus of the eye and the serum antibody titers were examined by the enzyme immunoassay shown below, and the spleen was excised 3 days after the final administration from a mouse showing a sufficient antibody titer. The spleen was cut to pieces in MEM (manufactured by Nissui Pharmaceutical), was loosened by using a pair of tweezers and centrifuged (1,200 rpm, 5 minutes), the resulting supernatant was discarded and the sediment was treated with Tris-ammonium chloride buffer (pH 7.65) for 1 to 2 minutes for eliminating erythrocytes, and then the remaining cells were washed three times with MEM and used for cell fusion.

### (3) Enzyme immunoassay

The MT4-MMP catalytic domain obtained in Example 2(1) was subjected to refolding and used as the antigen in the assay. Into a 96 well EIA plate (manufactured by Greiner), 10 µg/ml of the MT4-MMP catalytic domain prepared in the above was dispensed at 50 µl/well and allowed to stand at 4°C overnight for adsorption. After washing, 1% BSA/PBS (Dulbecco's phosphate buffered saline without magnesium and calcium) was added at 100 µl/well, and the reaction was carry out at room temperature for 1 hour to block the remaining active groups. After discarding 1% BSA/PBS, antiserum of immunized mouse, a culture supernatant of anti-MT4-MMP catalytic domain monoclonal antibody or purified monoclonal antibody was dispensed at 50 µl/well to carry out the reaction for 2 hours. After washing with 0.05% Tween 20/PBS, a peroxidase-labeled rabbit anti-mouse immunoglobulin (manufactured by DAKO) or peroxidase-labeled rabbit anti-rat immunoglobulin (manufactured by DAKO) was added at 50 µl/well to carry out the reaction at room temperature for 1 hour, the plate was washed with 0.05% Tween 20/PBS, and then color development was caused by using an ABTS substrate solution [2,2-azinobis(3-ethylbenzothiazole-6-sulfonic acid) ammonium], and the absorbance at OD 415 nm was measured by using a plate reader (NJ 2001; manufactured by Japan Intermed).

### (4) Preparation of mouse myeloma cells

The 8-azaguanine-resistant mouse myeloma cell strain P3-U1 was cultured in the normal medium and 2×10⁷ or more of the cells were secured at the time of cell fusion and used for cell fusion as a parent strain.

### (5) Preparation of hybridoma

The mouse spleen cells obtained in Example 2(2) and the myeloma cells obtained in Example 2(4) were mixed at a proportion of 10:1, the mixture was centrifuged (1,200 rpm, 5 minutes), the resulting supernatant was discarded, the thus precipitated cells were thoroughly disintegrated, a mixed solution comprising 2 g of polyethylene glycol-1,000 (PEG-1,000), 2 ml of MEM and 0.7 ml of dimethyl sulfoxide was added to the cells with stirring, in an amount of from 0.2 to 1 ml/10⁸ mouse spleen cells, at 37°C, 1 to 2 ml of MEM was added several times at 1- to 2-minute intervals, and then the total volume was adjusted to 50 ml by adding MEM. After centrifugation (900 rpm, 5 minutes), the supernatant was discarded, the resulting cells were loosened gently and then the cells were gently suspended in 100 ml of HAT medium by repeated sucking into and discharging from a measuring pipette.

The suspension was dispensed at 100 µl/well into a 96 well culture plate and incubated in a 5% CO₂ incubator at 37°C for 10 to 14 days. By examining the resulting culture supernatants by the enzyme immunoassay described in Example 2(3), wells which reacted with the MT4-MMP catalytic domain partial peptide but did not react with the control peptide were selected, and then cloning was repeated twice by changing the medium to HT medium and normal medium to establish anti-MT4-MMP monoclonal antibody producing hybridomas.

By the above method, 4 mouse monoclonal antibodies KM2892, KM2893, KM2894 and KM2895 were selected. Also, by applying the same method to rats, 11 rat monoclonal antibodies KM2896, KM2897, KM2898, KM2899, KM2900, KM2901, KM2903, KM2904, KM2906, KM2909 and KM2910 were selected.

### (6) Purification of monoclonal antibody

Each of the hybridomas obtained in Example 2(5) was intraperitoneally injected into 8-week-old female nude mice (Balb/c) treated with pristane at a dose of 5 to 20×10⁶ cells/head. The hybridoma caused ascites tumor in 10 to 21 days after the injection. The ascitic fluid was collected (1 to 8 ml/head) from the ascitic fluid-filled mice and centrifuged (3,000 rpm, 5 minutes) to remove solid matter. An IgG was purified by the caprylic acid precipitation method (*Antibodies*) and used as a purified monoclonal antibody.

The subclass of the antibody was determined by the enzyme immunoassay using a subclass typing kit (Table 1).

**Table 1**

| Animal species | Antibody name | Class |
|---|---|---|
| Mouse | KM2892 | IgG1 |
| | KM2893 | IgG2a |
| | KM2894 | IgA or IgE |
| | KM2895 | IgG2a |
| | | |
| Rat | KM2896 | IgG2b |
| | KM2897 | IgG2b |
| | KM2898 | IgG2b |
| | KM2899 | IgG2b |
| | KM2900 | IgG2b |
| | KM2901 | IgG2b |
| | KM2903 | IgG2b |
| | KM2904 | IgG2b |
| | KM2909 | IgG2b |
| | KM2910 | IgG2b |
| | KM2906 | IgG2b |

### (7) Reactivity with MT4-MMP catalytic domain (enzyme immunoassay)

The reactivity of anti-MT4-MMP monoclonal antibodies selected in Example 2(5) with the MT4-MMP catalytic domain was examined by the enzyme immunoassay shown in Example 2(3). A protein obtained by carrying out ultrasonic disintegration of *E*. *coli* (*E*. *coli* derived protein) was used as the control.

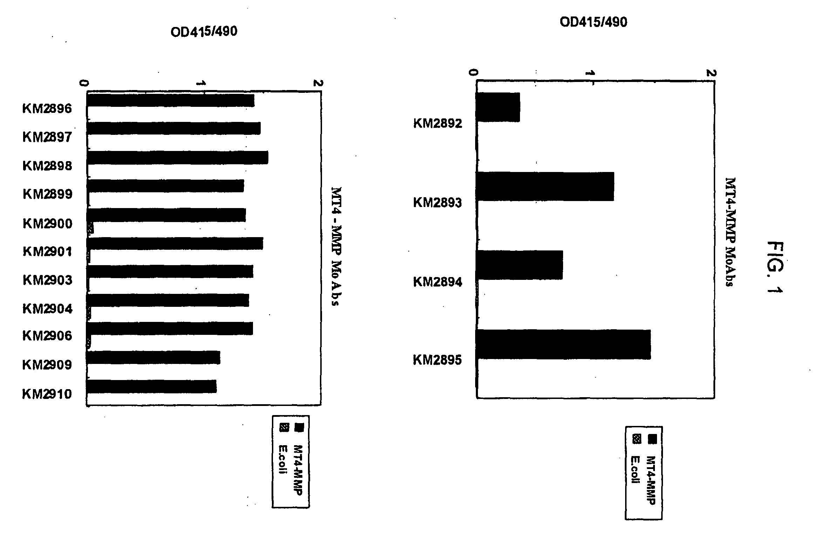

As shown in Fig. 1, the mouse monoclonal antibodies KM2892, KM2893, KM2894 and KM2895 and the rat monoclonal antibodies KM2896, KM2897, KM2898, KM2899, KM2900, KM2901, KM2903, KM2904, KM2906, KM2909 and KM2910, as anti-MT4-MMP monoclonal antibodies obtained by immunizing the MT4-MMP catalytic domain, specifically bound to the MT4-MMP catalytic domain.

### (8) Western blotting

Using the anti-MT4-MMP catalytic domain monoclonal antibodies obtained in Example 2(5), detection of the MT4-MMP protein by Western blotting was examined.

As the protein, a cell membrane fraction of an MT4-MMP gene-introduced COS-1 cell was used, and a cell membrane fraction of an MT5-MMP gene-introduced COS-1 cell as a control. Each of the gene-introduced cells was prepared by the method described in WO00/18805.

Each of the cell membrane fractions was fractionated by SDS-polyacrylamide electrophoresis *(Antibodies)* at 1.25×10² cells/lane and then blotted on a PVDF membrane. After blocking with 1% BSA/PBS, culture supernatant of each of the anti-MT4-MMP monoclonal antibodies was allowed to react at room temperature for 2 hours. After washing thoroughly with 0.05% Tween 20/PBS, a peroxidase-labeled anti-mouse immunoglobulin antibody (manufactured by DAKO) or a peroxidase-labeled anti-rat immunoglobulin antibody (manufactured by DAKO) was allowed to react as the secondary antibody at room temperature for 1 hour. After washing thoroughly with 0.05% Tween 20/PBS, each sample was detected using an ECL-detection kit (manufactured by Amersham) and exposed on an X-ray film. As a result, a band was detected at around 70 KDa corresponding to the molecular weight of MT4-MMP (shown by an arrow in the drawing) by the mouse monoclonal antibodies KM2892, KM2893, KM2894 and KM2895 and the rat monoclonal antibodies KM2896, KM2897, KM2898, KM2899, KM2900, KM2901, KM2903, KM2904, KM2906, KM2909 and KM2910, as anti-MT4-MMP monoclonal antibodies. On the other hand, all of these monoclonal antibodies did not show reactivity to MT5-MMP. Regarding an anti-PEG-treated granulocyte colony-stimulating factor monoclonal antibody KM511 (Japanese Published Unexamined Patent Application No. 165300/96; FERM BP-4880) which does not react with MT4-MMP and MT5-MMP, it did not specifically react with the band of around 70 KDa.

Based on the above results, it was shown that the anti-MT4-MMP catalytic domain monoclonal antibodies can detect the MT4-MMP protein on cells by Western blotting and can be applied to the diagnosis of various diseases relating to MT4-MMP such as inflammation and cancer.

### (9) Cell immunostaining

Using the anti-MT4-MMP catalytic domain monoclonal antibodies selected in Example 2(5), detection of the MT4-MMP protein expressing on cells by cell immunostaining was examined.

As the cells, COS-1 cell (ATCC No. CRL-1650), MT4-MMP gene-introduced COS-1 and MT5-MMP gene-introduced COS-1 were used. The MT4-MMP gene-introduced COS-1 and MT5-MMP gene-introduced COS-1 were produced by the method described in WO00/18805.

The cells were suspended in a mixed solution of trypsin and EDTA, washed with PBS and then, in order to improve antibody permeability of the cell membrane, treated with 100% methanol (ice-cooled) at 4°C for 10 minutes. After washing with PBS, they were blocked with 10 µg/ml human immunoglobulin (manufactured by Cappel) at room temperature for 30 minutes. The cells were dispensed at 1×10⁵ cells/tube and then centrifuged to discard the supernatant in each tube, and culture supernatant of each anti-MT4-MMP monoclonal antibody was added thereto and allowed to react at room temperature for 30 minutes.

After washing with PBS, an FITC-labeled anti-mouse immunoglobulin antibody (manufactured by Wako Pure Chemical Industries) was dispensed at 100 µl/tube and allowed to reaction at 4°C for 30 minutes in the dark. After thoroughly washing with PBS, they were analyzed by a cell analyzer (manufactured by Coulter; EPICS XLsystemII). The results are shown in Table 2.

**Table 2**

| mAb | Reactivity | | |
|---|---|---|---|
| | COS-1 | MT4-MMP/COS-1 | MT5-MMP/COS-1 |
| KM2892 | - | + | - |
| KM2893 | - | ++ | - |
| KM2894 | - | - | - |
| KM2895 | - | ++ | - |
| KM2896 | - | ++ | - |
| KM2897 | - | ++ | - |
| KM2898 | - | ++ | - |
| KM2899 | - | ++ | +w |
| KM2900 | - | + | - |
| KM2901 | - | ++ | - |
| KM2903 | - | ++ | - |
| KM2904 | - | ++ | - |
| KM2906 | - | ++ | +w |
| KM2909 | - | ++ | - |
| KM2910 | - | ++ | - |
| -: no reaction, +w: weak reaction, +: reaction, ++: strong reaction | | | |

The results show the respective reactions of cells of COS-1, MT4-MMP gene-introduced COS-1 and MT5-MMP gene-introduced COS-1 with monoclonal antibodies KM2892, KM2893, KM2894, KM2895, KM2896, KM2897, KM2898, KM2899, KM2900, KM2901, KM2903, KM2904, KM2906, KM2909 and KM2910, and the mouse IgG1 class anti-PEG-treated granulocyte colony-stimulating factor KM511 (Japanese Published Unexamined Patent Application No. 165300/96; FERM BP-4880) used as a negative control.

Reactivity with the MT4-MMP gene-introduced COS-1 cell was found in all of the anti-MT4-MMP monoclonal antibodies excluding the monoclonal antibody KM2894. Also, reactivity with the control COS-1 cell and MT5-MMP gene-introduced COS-1 cell was not found.

Thus, it was shown that the anti-MT4-MMP monoclonal antibodies can detect the MT4-MMP protein on cells by cell immunostaining and can be applied to the diagnosis of various diseases relating to MT4-MMP such as inflammation and cancer.

### Example 3

### Analysis of MT4-MMP on cell surface of hemocytic cell lines using flow cytometer:

The following analysis was carried out by using U937 (ATCC CRL-1593) as the human cell line.

A total of 2×10⁵ cells were put into a polyethylene tube (manufactured by Becton Dickinson Japan) and centrifuged at 1,500 rpm (400xg) for 3 minutes. After discarding the supernatant, 50 ml of a purified antibody anti-MT4-MMP antibody diluted to give a concentration of 5 mg/ml with 1% BSA/PBS was added. As the control antibody, a rat IgG2b antibody or mouse IgG2a antibody diluted to give a concentration of 5 mg/ml with 1% BSA/PBS was respectively used.

After stirring, the mixture was allowed to stand on ice for 30 minutes for reaction, adding 2 ml of cooled PBS was added thereto, followed by stirring, and then the mixture was centrifuged at 1,500 rpm (400×g) for 3 minutes to discard the supernatant (hereinafter, this operation is called "centrifugal washing"). As the secondary antibody, 50 ml of a biotinylated anti-mouse Ig antibody (manufactured by DAKO) or biotinylated anti-rat Ig antibody (manufactured by DAKO) diluted 100 folds with 1% BSA/PBS was added. After stirring, the mixture was allowed to stand on ice for 30 minutes. After washing by centrifugation, avidin (Streptavidin-FITC, manufactured by BD PharMingen) as a fluorescence label was diluted 200-fold with 1% BSA/PBS and 50 ml of the avidin dilution was further added, followed by stirring, and then mixture was allowed to stand on ice for 30 minutes. After washing by centrifugation, 500 ml of PBS was added thereto, followed by stirring, and then measurement was carried out by using FACScan (manufactured by Becton Dickinson Japan).

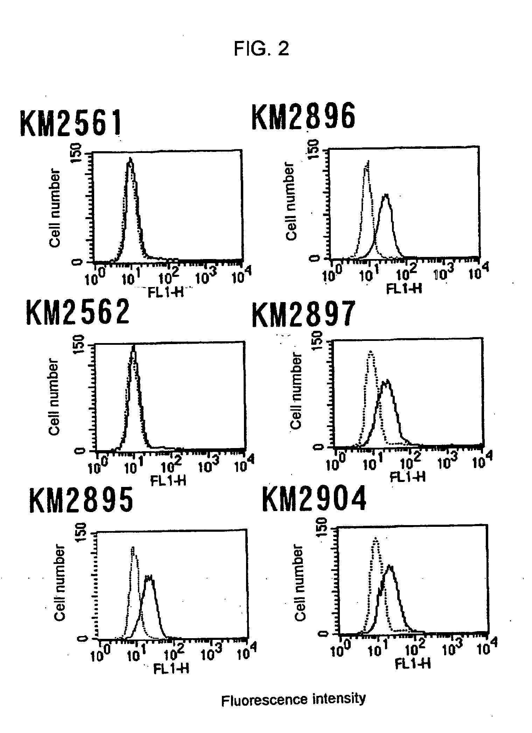

Results of the reactivity of antibodies to U937 cell are shown in Fig. 2. As shown in Fig. 2, the monoclonal antibodies KM2895, KM2896, KM2897 and KM2904 of the present invention reacted with the U 937 cell strongly. Based on the above results, it was considered that natural MT4-MMP on the cell membrane surface can be detected by using a monoclonal antibody which recognizes the MT4-MMP catalytic domain. On the other hand, the anti-MT4-MMP monoclonal antibodies KM2561 and KM2562 disclosed in WO00/18805 react with MT4-MMP transfectants which are considered to express large amount of MT4-MMP expression quantity (WO00/18805), but did not react with the cell surface MT4-MMP expressing in U937 cell.

Thus, it was shown that the anti-MT4-MMP catalytic domain monoclonal antibodies of the present invention can detect the MT4-MMP protein on cells by cell immunostaining and can be applied to diagnosis of various diseases relating to MT4-MMP such as inflammation and cancer.

### Example 4

### Examination of MT4-MMP determination system by sandwich ELISA:

Combination of monoclonal antibodies, which does not react with MT5-MMP but shows MT4-MMP-specific and high reactivity was examined by carrying out sandwich ELISA for all combinations of the anti-MT4-MMP monoclonal antibodies produced in Example 2.

To a 96 well flat bottom plate (Maxisorp, manufactured by Japan Intermed), 50 ml of each anti-MT4-MMP monoclonal antibody diluted to 5 mg/ml with PBS was added and allowed to stand at 4°C overnight for coating the plate (hereinafter, the anti-MT4-MMP monoclonal antibody is called "coating antibody"). After washing with PBS three times, 1% BSA/PBS was added at 200 ml/well and allowed to stand at room temperature for 2 to 4 hours to block the antibody. After removing 1% BSA/PBS, MT4-MMP refolded in advance, MT5-MMP activated with trypsin (WO00/18805) or the animal serum obtained in Example 5 was diluted to various concentrations with 1% BSA/PBS and dispensed at 50 ml into the wells to carry out the reaction at room temperature for 2 hours. After washing three times with 0.05% Tween 20/PBS, a rat anti-MT4-MMP monoclonal antibody diluted to 2 mg/ml with 1% BSA/PBS was added at 50 ml/well to carry out the reaction at room temperature for 1 hour (hereinafter, the rat anti-MT4-MMP monoclonal antibody is called "detecting antibody"). After washing with 0.005% Tween 20/PBS three times, a biotinylated anti-rat Ig (manufactured by DAKO, 1/4000 dilution) diluted with 1% BSA/PBS was added at 50 ml/well for reaction at room temperature for 1 hour. After washing with 0.05% Tween 20/PBS three times, horse radish peroxidase avidin D (manufactured by Vecter, 1/4000 dilution) diluted with 1% BSA/PBS was added at 50 ml/well and allowed to stand at room temperature for 20 minutes. After washing with 0.05% Tween 20/PBS three times, 3,3',5,5'-tetramethylbenzidine (TMB, manufactured by SIGMA-Aldrich) as the substrate of horse radish peroxidase avidin D was added at 100 ml/well for development of color at room temperature, and the reaction was stopped by adding 100 ml of 10% by volume H₂SO₄. Absorbance at a wavelength of 450 nm was measured by using an automatic plate reader EL340 (manufactured by Molecular Devices), and the data were calculated by Delta Soft (manufactured by Bio Metallics, Inc., Ver. 3-1.3B). The 4-parameter was used in the standard curve.

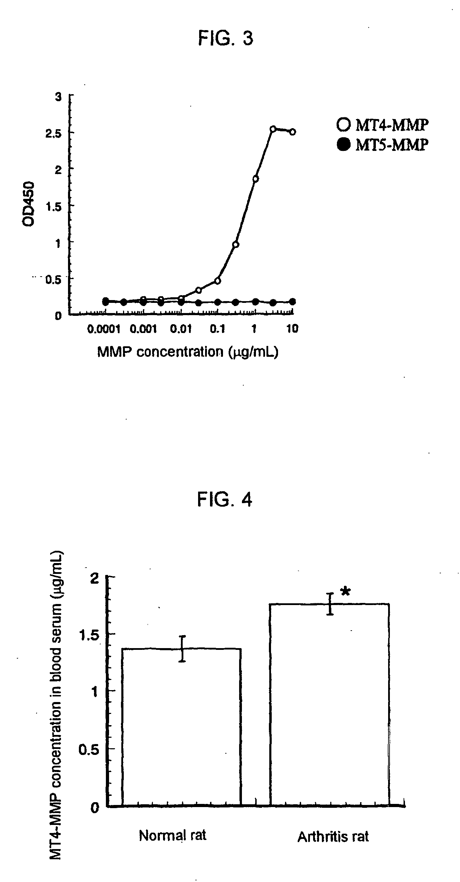

As shown in Fig. 3, it was able to specifically detect MT4-MMP within the concentration range of 10 to 5,000 ng/ml by the combination of KM2895 as the coating antibody with KM2904 as the detecting antibody.

Furthermore, MT4-MMP in a sample can be determined by using an MT4-MMP protein at known concentration as a standard sample.

### Example 5

### Determination of MT4-MMP in serum of rat collagen-induced arthritis:

### (1) Rat collagen-induced arthritis (CIA) model

A CIA model which is one of the rheumatoid arthritis models was prepared as follows.

Twelve-week-old DA rats (female, manufactured by Charles River Japan) were subjected to the test. A bovine joint-derived type II collagen (manufactured by Collagen Gijyutsu Kenshu Kai, or Collagen Techniques Training Club) was dissolved in 0.3% by volume acetic acid solution to give a concentration of 1 mg/ml and mixed with the same volume of incomplete Freund's adjuvant (hereinafter IFA, manufactured by DIFCO) under ice-cooling using Polytron (manufactured by KINEMATICA) to prepare an emulsion. The collagen was used by dissolving a freeze-dried preparation in the acetic acid solution when used.

The tail base areas of rats (n = 6) were shaved under ether anesthesia and 100 µl/site of the emulsified collagen solution was administered under the tail base skin using a 27 G injection needle for sensitization. At the same time, an untreated group was arranged and used as a normal group (n = 6).

### (2) Preparation of serum

On the 32nd day after the sensitization, blood samples were collected in SepaRapid Tube Mini S (manufactured by Sekisui Chemical) from the normal group and CIA-developed group, and serum samples were collected by centrifugation (Hitachi small type cooling centrifuge himac CF7D, rotor RT3S3, manufactured by Hitachi Koki) of the tubes at 3,000 revolutions per minute (1,500×g) at 4°C for 20 minutes.

### (3) Determination of MT4-MMP in sera of CIA model

Using the sandwich ELISA constructed in Example 4, expression of MT4-MMP in sera of the CIA model was detected. As a result of the detection of serum MT4-MMP, its significant increase was observed in the rat CIA in comparison with the untreated group (Fig. 4, p = 0.0195). A statistical analysis software SAS (Release 6.12, SAS Inc.) was used in the statistical analysis, and the significance test was carried out by Student's t-test.

MT4-MMP in serum and the like can also be carried out by sandwich ELISA using an MT4-MMP protein at known concentration as a standard sample.

### INDUSTRIAL APPLICABILITY

The present invention provides an anti-MT4-MMP monoclonal antibody which specifically reacts with MT4-MMP and can specifically detect or determine MT4-MMP protein by immunological assay. The anti-MT4-MMP monoclonal antibody of the present invention and a diagnosing kit which uses the same can provide highly sensitive and highly reliable detection in diagnosing various diseases relating to MT4-MMP. In addition, since it also reacts with MT4-MMP on cells, a medicament comprising the monoclonal antibody of the present invention is useful in treating various diseases relating to MT4-MMP.

### Free Text of Sequence

SEQ ID NO:1 - Explanation of synthetic sequence: synthetic DNA
SEQ ID NO:2 - Explanation of synthetic sequence: synthetic DNA
SEQ ID NO:3 - Explanation of synthetic sequence: synthetic DNA
SEQ ID NO:4 - Explanation of synthetic sequence: synthetic DNA

SEQUENCE LISTING

<110> KYOWA HAKKO KOGYO CO., LTD

<120> Monoclonal antibody against catalytic domain of MT4-MMP

<130> 11389W01

<140>
<141>

<150> JP2001-176256
<151> 2001-06-11

<160> 6

<170> PatentIn Ver. 2.0


<210> 1
<211> 32
<212> DNA
<213> Artificial Sequence
<220>
<223> Description of Artificial Sequence:synthetic DNA
<400> 1
ggatccatat gcaggctcca gcccccacca ag 32

<210> 2
<211> 34
<212> DNA
<213> Artificial Sequence
<220>
<223> Description of Artificial Sequence:synthetic DNA
<400> 2
ggcggccgct tacacagact cccgcacacc gtac 34

<210> 3
<211> 20
<212> DNA

<213> Artificial Sequence
<220>
<223> Description of Artificial Sequence:synthetic DNA
<400> 3
gctcggatcc actagtaacg                                                    20

<210> 4
<211> 17
<212> DNA
<213> Artificial Sequence
<220>
<223> Description of Artificial Sequence:synthetic DNA
<400> 4
gtgtgatgga tatctgc                                                       17

<210> 5
<211> 2423
<212> DNA
<213> Homo sapiens

<400> 5
ccggcggggg cgccgcggag agcggagggc gccgggctgc ggaacgcgaa gcggagggcg 60

cgggaccctg cacgccgccc gcgggcccat gtgagcgcc atg cgg cgc cgc gca    114
                                          Met Arg Arg Arg Ala
                                          1               5

gcc cgg gga ccc ggc ccg ccg ccc cca ggg ccc gga ctc tcg cgg ctg   162
Ala Arg Gly Pro Gly Pro Pro Pro Pro Gly Pro Gly Leu Ser Arg Leu
                10                  15                  20

ccg ctg ctg ccg ctg ccg ctg ctg ctg ctg ctg gcg ctg ggg acc cgc   210
Pro Leu Leu Pro Leu Pro Leu Leu Leu Leu Leu Ala Leu Gly Thr Arg
            25                  30                  35

ggg ggc tgc gcc gcg ccg gaa ccc gcg cgg cgc gcc gag gac ctc agc   258
Gly Gly Cys Ala Ala Pro Glu Pro Ala Arg Arg Ala Glu Asp Leu Ser
        40                  45                  50

ctg gga gtg gag tgg cta agc agg ttc ggt tac ctg ccc ccg gct gac   306
Leu Gly Val Glu Trp Leu Ser Arg Phe Gly Tyr Leu Pro Pro Ala Asp

55                  60                  65

ccc aca aca ggg cag ctg cag acg caa gag gag ctg tct aag gcc atc     354
Pro Thr Thr Gly Gln Leu Gln Thr Gln Glu Glu Leu Ser Lys Ala Ile
 70                  75                  80                  85

aca gcc atg cag cag ttt ggt ggc ctg gag gcc acc ggc atc ctg gac     402
Thr Ala Met Gln Gln Phe Gly Gly Leu Glu Ala Thr Gly Ile Leu Asp
                 90                  95                 100

gag gcc acc ctg gcc ctg atg aaa acc cca cgc tgc tcc ctg cca gac     450
Glu Ala Thr Leu Ala Leu Met Lys Thr Pro Arg Cys Ser Leu Pro Asp
            105                 110                 115

ctc cct gtc ctg acc cag gct cgc agg aga cgc cag gct cca gcc ccc     498
Leu Pro Val Leu Thr Gln Ala Arg Arg Arg Arg Gln Ala Pro Ala Pro
        120                 125                 130

acc aag tgg aac aag agg aac ctg tcg tgg agg gtc cgg acg ttc cca     546
Thr Lys Trp Asn Lys Arg Asn Leu Ser Trp Arg Val Arg Thr Phe Pro
    135                 140                 145

cgg gac tca cca ctg ggg cac gac acg gtg cgt gca ctc atg tac tac     594
Arg Asp Ser Pro Leu Gly His Asp Thr Val Arg Ala Leu Met Tyr Tyr
150                 155                 160                 165

gcc ctc aag gtc tgg agc gac att gcg ccc ctg aac ttc cac gag gtg     642
Ala Leu Lys Val Trp Ser Asp Ile Ala Pro Leu Asn Phe His Glu Val
                170                 175                 180

gcg ggc agc acc gcc gac atc cag atc gac ttc tcc aag gcc gac cat     690
Ala Gly Ser Thr Ala Asp Ile Gln Ile Asp Phe Ser Lys Ala Asp His
            185                 190                 195

aac gac ggc tac ccc ttc gac gcc cgg cgg cac cgt gcc cac gcc ttc     738
Asn Asp Gly Tyr Pro Phe Asp Ala Arg Arg His Arg Ala His Ala Phe
        200                 205                 210

ttc ccc ggc cac cac cac acc gcc ggg tac acc cac ttt aac gat gac     786
Phe Pro Gly His His His Thr Ala Gly Tyr Thr His Phe Asn Asp Asp
    215                 220                 225

gag gcc tgg acc ttc cgc tcc tcg gat gcc cac ggg atg gac ctg ttt 834
Glu Ala Trp Thr Phe Arg Ser Ser Asp Ala His Gly Met Asp Leu Phe
230                 235                 240                 245

gca gtg gct gtc cac gag ttt ggc cac gcc att ggg tta agc cat gtg 882
Ala Val Ala Val His Glu Phe Gly His Ala Ile Gly Leu Ser His Val
                250                 255                 260

gcc gct gca cac tcc atc atg cgg ccg tac tac cag ggc ccg gtg ggt 930
Ala Ala Ala His Ser Ile Met Arg Pro Tyr Tyr Gln Gly Pro Val Gly
            265                 270                 275

gac ccg ctg cgc tac ggg ctc ccc tac gag gac aag gtg cgc gtc tgg 978
Asp Pro Leu Arg Tyr Gly Leu Pro Tyr Glu Asp Lys Val Arg Val Trp
        280                 285                 290

cag ctg tac ggt gtg cgg gag tct gtg tct ccc acg gcg cag ccc gag 1026
Gln Leu Tyr Gly Val Arg Glu Ser Val Ser Pro Thr Ala Gln Pro Glu
    295                 300                 305

gag cct ccc ctg ctg ccg gag ccc cca gac aac cgg tcc agc gcc ccg 1074
Glu Pro Pro Leu Leu Pro Glu Pro Pro Asp Asn Arg Ser Ser Ala Pro
310                 315                 320                 325

ccc agg aag gac gtg ccc cac aga tgc agc act cac ttt gac gcg gtg 1122
Pro Arg Lys Asp Val Pro His Arg Cys Ser Thr His Phe Asp Ala Val
                330                 335                 340

gcc cag atc cgg ggt gaa gct ttc ttc ttc aaa ggc aag tac ttc tgg 1170
Ala Gln Ile Arg Gly Glu Ala Phe Phe Phe Lys Gly Lys Tyr Phe Trp
            345                 350                 355

cgg ctg acg cgg gac cgg cac ctg gtg tcc ctg cag ccg gca cag atg 1218
Arg Leu Thr Arg Asp Arg His Leu Val Ser Leu Gln Pro Ala Gln Met
        360                 365                 370

cac cgc ttc tgg cgg ggc ctg ccg ctg cac ctg gac agc gtg gac gcc 1266
His Arg Phe Trp Arg Gly Leu Pro Leu His Leu Asp Ser Val Asp Ala
    375                 380                 385

gtg tac gag cgc acc agc gac cac aag atc gtc ttc ttt aaa gga gac 1314
Val Tyr Glu Arg Thr Ser Asp His Lys Ile Val Phe Phe Lys Gly Asp
390                 395                 400                 405

agg tac tgg gtg ttc aag gac aat aac gta gag gaa gga tac ccg cgc 1362
Arg Tyr Trp Val Phe Lys Asp Asn Asn Val Glu Glu Gly Tyr Pro Arg
                410                 415                 420

ccc gtc tcc gac ttc agc ctc ccg cct ggc ggc atc gac gct gcc ttc 1410
Pro Val Ser Asp Phe Ser Leu Pro Pro Gly Gly Ile Asp Ala Ala Phe
            425                 430                 435

tcc tgg gcc cac aat gac agg act tat ttc ttt aag gac cag ctg tac 1458
Ser Trp Ala His Asn Asp Arg Thr Tyr Phe Phe Lys Asp Gln Leu Tyr
        440                 445                 450

tgg cgc tac gat gac cac acg agg cac atg gac ccc ggc tac ccc gcc 1506
Trp Arg Tyr Asp Asp His Thr Arg His Met Asp Pro Gly Tyr Pro Ala
    455                 460                 465

cag agc ccc ctg tgg agg ggt gtc ccc agc acg ctg gac gac gcc atg 1554
Gln Ser Pro Leu Trp Arg Gly Val Pro Ser Thr Leu Asp Asp Ala Met
470                 475                 480                 485

cgc tgg tcc gac ggt gcc tcc tac ttc ttc cgt ggc cag gag tac tgg 1602
Arg Trp Ser Asp Gly Ala Ser Tyr Phe Phe Arg Gly Gln Glu Tyr Trp
                490                 495                 500

aaa gtg ctg gat ggc gag ctg gag gtg gca ccc ggg tac cca cag tcc 1650
Lys Val Leu Asp Gly Glu Leu Glu Val Ala Pro Gly Tyr Pro Gln Ser
            505                 510                 515

acg gcc cgg gac tgg ctg gtg tgt gga gac tca cag gcc gat gga tct 1698
Thr Ala Arg Asp Trp Leu Val Cys Gly Asp Ser Gln Ala Asp Gly Ser
        520                 525                 530

gtg gct gcg ggc gtg gac gcg gca gag ggg ccc cgc gcc cct cca gga 1746
Val Ala Ala Gly Val Asp Ala Ala Glu Gly Pro Arg Ala Pro Pro Gly
    535                 540                 545

caa cat gac cag agc cgc tcg gag gac ggt tac gag gtc tgc tca tgc 1794

Gln His Asp Gln Ser Arg Ser Glu Asp Gly Tyr Glu Val Cys Ser Cys
550                 555                 560                 565

acc tct ggg gca tcc tct ccc ccg ggg gcc cca ggc cca ctg gtg gct    1842
Thr Ser Gly Ala Ser Ser Pro Pro Gly Ala Pro Gly Pro Leu Val Ala
                570                 575                 580

gcc acc atg ctg ctg ctg ctg ccg cca ctg tca cca ggc gcc ctg tgg    1890
Ala Thr Met Leu Leu Leu Leu Pro Pro Leu Ser Pro Gly Ala Leu Trp
            585                 590                 595

aca gcg gcc cag gcc ctg acg cta tgacacacag cgcgagccca tgagaggaca    1944
Thr Ala Ala Gln Ala Leu Thr Leu
        600                 605

gaggcggtgg gacagcctgg ccacagaggg caaggactgt gccggagtcc ctgggggagg 2004

tgctggcgcg ggatgaggac gggccaccct ggcaccggaa ggccagcaga gggcacggcc 2064

cgccagggct gggcaggctc aggtggcaag gacggagctg tcccctagtg agggactgtg 2124

ttgactgacg agccgagggg tggccgctcc agaagggtgc ccagtcaggc cgcaccgccg 2184

ccagcctcct ccggccctgg agggagcatc tcgggctggg ggcccacccc tctctgtgcc 2244

ggcgccacca accccaccca cactgctgcc tggtgctccc gccggcccac agggcctccg 2304

tccccaggtc cccagtgggg cagccctccc cacagacgag ccccccacat ggtgccgcgg 2364

cacgtccccc ctgtgacgcg ttccagacca acatgacctc tccctgcttt gtagcggcc  2423

<210> 6
<211> 605
<212> PRT
<213> Homo sapiens

<400> 6
Met Arg Arg Arg Ala Ala Arg Gly Pro Gly Pro Pro Pro Pro Gly Pro
1               5                   10                  15

Gly Leu Ser Arg Leu Pro Leu Leu Pro Leu Pro Leu Leu Leu Leu

20                   25                   30

Ala Leu Gly Thr Arg Gly Gly Cys Ala Ala Pro Glu Pro Ala Arg Arg
        35                  40                  45

Ala Glu Asp Leu Ser Leu Gly Val Glu Trp Leu Ser Arg Phe Gly Tyr
    50                  55                  60

Leu Pro Pro Ala Asp Pro Thr Thr Gly Gln Leu Gln Thr Gln Glu Glu
65                  70                  75                  80

Leu Ser Lys Ala Ile Thr Ala Met Gln Gln Phe Gly Gly Leu Glu Ala
                85                  90                  95

Thr Gly Ile Leu Asp Glu Ala Thr Leu Ala Leu Met Lys Thr Pro Arg
            100                 105                 110

Cys Ser Leu Pro Asp Leu Pro Val Leu Thr Gln Ala Arg Arg Arg Arg
        115                 120                 125

Gln Ala Pro Ala Pro Thr Lys Trp Asn Lys Arg Asn Leu Ser Trp Arg
    130                 135                 140

Val Arg Thr Phe Pro Arg Asp Ser Pro Leu Gly His Asp Thr Val Arg
145                 150                 155                 160

Ala Leu Met Tyr Tyr Ala Leu Lys Val Trp Ser Asp Ile Ala Pro Leu
                165                 170                 175

Asn Phe His Glu Val Ala Gly Ser Thr Ala Asp Ile Gln Ile Asp Phe
            180                 185                 190

Ser Lys Ala Asp His Asn Asp Gly Tyr Pro Phe Asp Ala Arg Arg His
        195                 200                 205

Arg Ala His Ala Phe Phe Pro Gly His His His Thr Ala Gly Tyr Thr
    210                 215                 220

His Phe Asn Asp Asp Glu Ala Trp Thr Phe Arg Ser Ser Asp Ala His
225                 230                 235                 240

Gly Met Asp Leu Phe Ala Val Ala Val His Glu Phe Gly His Ala Ile
                245                 250                 255

Gly Leu Ser His Val Ala Ala Ala His Ser Ile Met Arg Pro Tyr Tyr
            260                 265                 270

Gln Gly Pro Val Gly Asp Pro Leu Arg Tyr Gly Leu Pro Tyr Glu Asp
        275                 280                 285

Lys Val Arg Val Trp Gln Leu Tyr Gly Val Arg Glu Ser Val Ser Pro
    290                 295                 300

Thr Ala Gln Pro Glu Glu Pro Pro Leu Leu Pro Glu Pro Pro Asp Asn
305                 310                 315                 320

Arg Ser Ser Ala Pro Pro Arg Lys Asp Val Pro His Arg Cys Ser Thr
                325                 330                 335

His Phe Asp Ala Val Ala Gln Ile Arg Gly Glu Ala Phe Phe Phe Lys
            340                 345                 350

Gly Lys Tyr Phe Trp Arg Leu Thr Arg Asp Arg His Leu Val Ser Leu
        355                 360                 365

Gln Pro Ala Gln Met His Arg Phe Trp Arg Gly Leu Pro Leu His Leu
    370                 375                 380

Asp Ser Val Asp Ala Val Tyr Glu Arg Thr Ser Asp His Lys Ile Val
385                 390                 395                 400

Phe Phe Lys Gly Asp Arg Tyr Trp Val Phe Lys Asp Asn Asn Val Glu
                405                 410                 415

Glu Gly Tyr Pro Arg Pro Val Ser Asp Phe Ser Leu Pro Pro Gly Gly
            420                 425                 430

Ile Asp Ala Ala Phe Ser Trp Ala His Asn Asp Arg Thr Tyr Phe Phe
        435                 440                 445

Lys Asp Gln Leu Tyr Trp Arg Tyr Asp Asp His Thr Arg His Met Asp
    450                 455                 460

Pro Gly Tyr Pro Ala Gln Ser Pro Leu Trp Arg Gly Val Pro Ser Thr
465 470 475 480

Leu Asp Asp Ala Met Arg Trp Ser Asp Gly Ala Ser Tyr Phe Phe Arg
485 490 495

Gly Gln Glu Tyr Trp Lys Val Leu Asp Gly Glu Leu Glu Val Ala Pro
                500                 505                 510

Gly Tyr Pro Gln Ser Thr Ala Arg Asp Trp Leu Val Cys Gly Asp Ser
            515                 520                 525

Gln Ala Asp Gly Ser Val Ala Ala Gly Val Asp Ala Ala Glu Gly Pro
        530                 535                 540

Arg Ala Pro Pro Gly Gln His Asp Gln Ser Arg Ser Glu Asp Gly Tyr
545 550 555 560

Glu Val Cys Ser Cys Thr Ser Gly Ala Ser Ser Pro Pro Gly Ala Pro
565 570 575

Gly Pro Leu Val Ala Ala Thr Met Leu Leu Leu Leu Pro Pro Leu Ser
                580                 585                 590

Pro Gly Ala Leu Trp Thr Ala Ala Gln Ala Leu Thr Leu
595 600 605

INTERNATIONAL FORM

BUDAPEST TREATY ON THE INTERNATIO-
NAL RECOGNITION OF THE DEPOSIT OF
MICROORGANISMS FOR THE PURPOSES OF
PATENT PROCEDURE

RECEIPT IN THE CASE OF AN ORIGINAL
DEPOSIT
issued pursuant to Rule 7.1 by the
INTERNATIONAL DEPOSITARY AUTHORITY
identified at the bottom of this
page.

Name (Appellation) | Kyowa Hakko Kogyo Co., Ltd.
Depositor | President, Tadashi HIRATA
Address 〒 | 6-1, Ohtematchi 1-chome, Chiyoda-ku, Tokyo

1. Indication of Microorganism
(Indication for identification used by the depositor) | (Accession No.)
KM2895 | FERM BP-7601
2. Scientific Property and Taxonomic Designation
To the microorganism described in column 1, document(s) which describe(s) the
following item(s) are attached
☒ Scientific Property
☒ Taxonomic Designation
3. Receipt and Acceptance
The International Depositary Authority accepts the microorganism described
in column 1 received on May 24, 2001 (date of the original deposit)
4. Receipt of Request for Transfer
The International Depositary Authority received the microorganism described in
column 1 received on ______ (date of the original deposit), and then received a
request for transfer to deposit based on the Budapest Treaty from the original
deposit on ______.
5. International Depositary Authority
Appellation: International Patent Organism Department
National Instituted of Advanced Industrial Science and Technology

Dr. Yasuhiko Komatsu, Director (seal)

Address: AIST Tsukuba Central 6,1-1, Higashi 1-Chome Tsukuba-shi,
Ibaraki-ken 305-8566, JAPAN

May 24, 2001

INTERNATIONAL FORM

BUDAPEST TREATY ON THE INTERNATIO-
NAL RECOGNITION OF THE DEPOSIT OF
MICROORGANISMS FOR THE PURPOSES OF
PATENT PROCEDURE

RECEIPT IN THE CASE OF AN ORIGINAL
DEPOSIT
issued pursuant to Rule 7.1 by the
INTERNATIONAL DEPOSITARY AUTHORITY
identified at the bottom of this
page.

Name (Appellation) | Kyowa Hakko Kogyo Co., Ltd.
Depositor | President, Tadashi HIRATA
Address | 〒 6-1, Ohtematchi 1-chome, Chiyoda-ku, Tokyo

1. Indication of Microorganism
(Indication for identification used by the depositor) | (Accession No.)
KM2904 | FERM BP-7602
2. Scientific Property and Taxonomic Designation
To the microorganism described in column 1, document(s) which describe(s) the
following item(s) are attached.
☒ Scientific Property
☒ Taxonomic Designation
3. Receipt and Acceptance
The International Depositary Authority accepts the microorganism described
in column 1 received on May 24, 2001 (date of the original deposit)
4. Receipt of Request for Transfer
The International Depositary Authority received the microorganism described in
column 1 received on ______ (date of the original deposit), and then received a
request for transfer to deposit based on the Budapest Treaty from the original
deposit on ______.
5. International Depositary Authority
Appellation: International Patent Organism Department
National Instituted of Advanced Industrial Science and Technology

Dr. Yasuhiko Komatsu, Director (seal)

Address: AIST Tsukuba Central 6,1-1, Higashi 1-Chome Tsukuba-shi,
Ibaraki-ken 305-8566, JAPAN

May 24, 2001

## Claims

1. A monoclonal antibody which specifically binds to an MT4-MMP catalytic domain.

2. The monoclonal antibody according to claim 1, wherein the MT4-MMP catalytic domain is an amino acid sequence comprising 128th to 296th positions in the amino acid sequence represented by SEQ ID NO:6.

3. The monoclonal antibody according to claim 1 or 2, wherein the monoclonal antibody is a monoclonal antibody produced by a hybridoma selected from the group consisting of hybridoma KM2895, hybridoma KM2896, hybridoma KM2897 and hybridoma KM2904.

4. A hybridoma which produces the monoclonal antibody according to any one of claims 1 to 3.

5. The hybridoma according to claim 4, wherein the hybridoma is a hybridoma selected from the group consisting of hybridoma KM2895, hybridoma KM2896, hybridoma KM2897 and hybridoma KM2904.

6. The monoclonal antibody according to any one of claims 1 to 3, wherein the monoclonal antibody is a recombinant antibody.

7. The monoclonal antibody according to claim 6, wherein the recombinant antibody is a monoclonal antibody selected from a humanized antibody and an antibody fragment.

8. The monoclonal antibody according to claim 7, wherein the humanized antibody is a human chimeric antibody.

9. The human chimeric antibody according to claim 8, which comprises an antibody heavy chain (H chain) variable region (V region) and an antibody light chain (L chain) V region of the monoclonal antibody according to any one of claims 1 to 3 and an H chain constant region (C region) and an L chain C region of a human antibody.

10. The human chimeric antibody according to claim 9, wherein the amino acid sequences of the H chain V region and L chain V region have the same amino acid sequences of the H chain V region and L chain V region, respectively, of a monoclonal antibody selected from the group consisting of monoclonal antibodies KM2895, KM2896, KM2897 and KM2904.

11. The monoclonal antibody according to claim 7, wherein the humanized antibody is a complementarity determining region-grafted antibody (CDR-grafted antibody).

12. The CDR-grafted antibody according to claim 11, which comprises complementarity determining regions of H chain and L chain V regions of the monoclonal antibody according to any one of claims 1 to 3 and H chain and L chain C regions and a framework region of a V region of a human antibody.

13. The CDR-grafted antibody according to claim 12, wherein the amino acid sequences of the H chain V region and the L chain V region have the same amino acid sequences of an H chain V region and an L chain V region, respectively, of a monoclonal antibody selected from the group consisting of monoclonal antibodies KM2895, KM2896, KM2897 and KM2904.

14. The monoclonal antibody according to claim 7, wherein the antibody fragment is an antibody selected from the group consisting of Fab, Fab', F(ab')₂, a single chain antibody and a disulfide-stabilized antibody.

15. The single chain antibody according to claim 14, which comprises an H chain V region and an L chain V region of the monoclonal antibody according to any one of claims 1 to 3.

16. The single chain antibody according to claim 15, wherein the amino acid sequences of the H chain V region and the L chain V region of the single chain antibody have the same amino acid sequences of an H chain V region and an L chain V region, respectively, of a monoclonal antibody selected from the group consisting of monoclonal antibodies KM2895, KM2896, KM2897 and KM2904.

17. The disulfide-stabilized antibody according to claim 14, which comprises an H chain V region and an L chain and V region of the monoclonal antibody according to any one of claims 1 to 3.

18. The disulfide-stabilized antibody according to claim 17, wherein the amino acid sequences of the H chain V region and the L chain V region of the disulfide-stabilized antibody have the same amino acid sequences of an H chain V region and an L chain V region, respectively, of a monoclonal antibody selected from the group consisting of monoclonal antibodies KM2895, KM2896, KM2897 and KM2904.

19. The monoclonal antibody according to any one of claims 1 to 3 and 6 to 18, wherein the monoclonal antibody is a fusion antibody linked with an agent chemically or genetically.

20. A method for immunologically detecting an MT4-MMP catalytic subunit, which comprises using the monoclonal antibody according to any one of claims 1 to 3 and 6 to 19.

21. The method according to claim 20, wherein the immunologically detecting method is selected from the group consisting of immunoassay, Western blotting, immunohistochemical staining, cell immunostaining and dot blotting.

22. A method for immunologically determining an MT4-MMP catalytic subunit, which comprises using the monoclonal antibody according to any one of claims 1 to 3 and 6 to 19.

23. The method according to claim 22, wherein the immunologically detecting method is a method selected from the group consisting of immunoassay, Western blotting, immunohistochemical staining, cell immunostaining and dot blotting.

24. A method for diagnosing diseases relating to MT4-MMP, which comprises using the monoclonal antibody according to any one of claims 1 to 3 and 6 to 19.

25. The diagnostic method according to claim 24, wherein the disease relating to MT4-MMP is rheumatoid arthritis.

26. An agent for diagnosing disease relating to MT4-MMP, which comprises the monoclonal antibody according to any one of claims 1 to 3 and 6 to 19 as an active ingredient.

27. The diagnostic agent according to claim 26, wherein the disease relating to MT4-MMP is rheumatoid arthritis.

28. A therapeutic agent for treating diseases relating to MT4-MMP, which comprises the monoclonal antibody according to any one of claims 1 to 3 and 6 to 19 as an active ingredient.

29. The therapeutic agent according to claim 28, wherein the disease relating to MT4-MMP is rheumatoid arthritis.

30. A reagent which comprises the monoclonal antibody according to any one of claims 1 to 3 and 6 to 19.

31. A kit for detecting diseases relating to MT4-MMP, which comprises the reagent according to claim 30.

32. The kit according to claim 31, wherein the disease relating to MT4-MMP is rheumatoid arthritis.
